(19)

**Europäisches Patentamt**

**European Patent Office**

**Office européen des brevets**

(11) **EP 4 591 787 A1**

(12) **EUROPEAN PATENT APPLICATION**

(43) Date of publication:
30.07.2025 Bulletin 2025/31

(21) Application number: 25154192.6

(22) Date of filing: 27.01.2025

(51) International Patent Classification (IPC):
*A61B 5/0535* (2021.01) *A61B 5/087* (2006.01)

(52) Cooperative Patent Classification (CPC):
**A61B 5/0816; A61B 5/0535; A61B 5/1135;**
**A61B 5/6823; A61B 5/7275;** A61B 5/087

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: 26.01.2024 US 202463625787 P
26.01.2024 US 202463625749 P
26.01.2024 US 202463625741 P
09.02.2024 US 202463551957 P
09.02.2024 US 202463551956 P
09.02.2024 US 202463551972 P

(71) Applicant: **Analog Devices International**
**Unlimited Company**
**Co. Limerick (IE)**

(72) Inventors:
• **GOPINATHAN, Venugopal**
**Wilmington, 01887 (US)**
• **DUTTA, Goutam**
**Limerick (IE)**
• **BASAK, Dibyajyoti**
**Limerick (IE)**
• **GANESAN, Sriram**
**Limerick (IE)**
• **AKL, Tony**
**Wilmington, 01887 (US)**

(74) Representative: **Horler, Philip John**
**Withers & Rogers LLP**
**2 London Bridge**
**London SE1 9RA (GB)**

(54) **ASSESSMENT OF AIRWAY CONSTRICTION BASED ON TRENDS OF RATIO OF EXHALATION TIME AND INHALATION TIME**

(57) Technologies are provided for monitoring of status changes of a cardiopulmonary condition. In some cases, a method includes: receiving, thoracic impedance (TI) measurement signals corresponding to a subject, the TI measurement signals being time-dependent and obtained during a time interval; generating, using the TI measurement signals, respiration signals corresponding to the subject during the time interval; determining, over the time interval, using the respiration signals, multiple values of a ratio of an exhalation time and an inhalation time of the subject; monitoring, over the time interval, using the multiple values, a time-dependence of the ratio; and identifying, based on the time-dependence, a status change of a cardiopulmonary condition of the subject.

EP 4 591 787 A1

**Description**

**CROSS-REFERENCE TO RELATED APPLICATIONS**

This application claims the benefit of and priority to U.S. Provisional Patent Application No. 63/625,749, filed January 26, 2024, U.S. Provisional Patent Application No. 63/625,741, filed January 26, 2024, U.S. Provisional Patent Application No. 63/625,787, filed January 26, 2024, U.S. Provisional Patent Application No. 63/551,972, filed February 9, 2024, U.S. Provisional Patent Application No. 63/551,956, filed February 9, 2024, and U.S. Provisional Patent Application No. 63/551,957, filed February 9, 2024, the contents of each of which applications are hereby incorporated by reference herein in their entireties.

**BACKGROUND**

Cardiopulmonary conditions are chronic medical conditions that include chronic obstructive pulmonary disease (COPD) and congestive heart failure (CHF). In particular, COPD involves a combination of emphysema and chronic bronchitis, where the chronic bronchitis can have one of various degrees of severity. Even when treated, cardiopulmonary conditions can deteriorate over time. As a consequence, depending on the magnitude of the deterioration, a subject afflicted by the condition may undergo significant medical intervention. In addition, patients with more significant deterioration exhibit more episodes of exacerbation. Accordingly, monitoring the status of a cardiopulmonary condition can help avoiding medical intervention. Monitoring the status of a cardiopulmonary condition, however, typically involves ambulatory care. While the status of the cardiopulmonary condition may be updated regularly by a clinician during routine visits to a health services facility, the intervening time between visits and updates can result in the deterioration of the cardiopulmonary condition without appropriate treatment. Further, a subject afflicted by a cardiopulmonary condition may have difficulty adhering to a long-term routine of ambulatory care to monitor the status of the cardiopulmonary condition. A consequence of poor adherence can lead to identification of a status change of the cardiopulmonary condition after a prolonged deterioration of the cardiopulmonary condition or when the subject is in distress. Both situations being clearly undesirable and detrimental to favorable prognosis.

Therefore, several technical challenges remain in the field of monitoring cardiopulmonary conditions and the technologies for such monitoring. Improved technologies that address those challenges are desirable.

**SUMMARY**

Embodiments of the disclosure address the issue of identification of changes in status of a cardiopulmonary condition of a subject.

In some aspects, the disclosure provides a system. The system includes: a wearable device configured to be mounted on a torso of a subject, the wearable device including multiple electrodes and multiple sensor devices, wherein each one of the multiple electrodes is configured to contact a respective skin area at a respective skin location on the torso of the subject, and wherein the multiple sensor devices include: an electric sensor device functionally coupled with the multiple electrodes and configured to generate first measurement signals during a measurement period; and a movement sensor device configured to generate second measurement signals during the measurement period; and a computing device configured to communicate with the wearable device and configured to: receive the first measurement signals and the second measurement signals; and determine, using a combination of the first measurement signals and the second measurement signals, a value of a metric or respective values in a combination of metrics, wherein each one of the metric and the combination of metrics is indicative of status of a cardiopulmonary condition of the subject.

In some aspects, the disclosure provides a method. The method includes: generating, by one or more processors, individually or in combination, using thoracic impedance (TI) measurement signals during a time interval, respiration signals corresponding to a subject, wherein the TI measurement signals are time-dependent; generating, by the one or more processors, individually or in combination, using acceleration measurement signals during the time interval, movement signals corresponding to movement of a chest wall of the subject, wherein the acceleration measurement signals are time-dependent; determining, by the one or more processors, individually or in combination, over the time interval, using the respiration signals and the movement signals, multiple values of a metric associated with respiration of the subject; monitoring, by the one or more processors, individually or in combination, over the time interval, using the multiple values, a time-dependence of the metric; and identifying, by the one or more processors, individually or in combination, based on the time-dependence, a status change of a cardiopulmonary condition of the subject.

In some aspects, the disclosure provides a computing system. The computing system includes: at least one processor; and at least one memory device storing processor-executable instructions that, in response to execution by the at least one processor, individually or in combination, cause the computing system at least to: generate, using thoracic impedance (TI) measurement signals during a time interval, respiration signals corresponding to a subject, wherein the TI measurement signals are time-dependent; generate, using acceleration measurement signals during the time interval, movement signals corresponding to movement of a chest wall of the subject, wherein the acceleration measurement signals are time-dependent; determine, over the time interval, using the respiration signals and the movement signals, multiple values of a metric associated with respiration of the subject; monitor, over the time interval, using the multiple values, a time-dependence of the metric; and identify, based on the time-dependence, a status change of a cardiopulmonary condition of the subject.

In some aspects, the disclosure provides a user device. The user interface includes: at least one processor; and at least one memory device storing processor-executable instructions that, in response to execution by the at least one processor, individually or in combination, cause the user device at least to: generate, using thoracic impedance (TI) measurement signals during a time interval, respiration signals corresponding to a subject, wherein the TI measurement signals are time-dependent; generate, using acceleration measurement signals during the time interval, movement signals corresponding to movement of a chest wall of the subject, wherein the acceleration measurement signals are time-dependent; determine, over the time interval, using the respiration signals and the movement signals, multiple values of a metric associated with respiration of the subject; monitor, over the time interval, using the multiple values, a time-dependence of the metric; and identify, based on the time-dependence, a status change of a cardiopulmonary condition of the subject.

In some aspects, the disclosure provides a method. The method includes: receiving, by one or more processors, individually or in combination, thoracic impedance (TI) measurement signals corresponding to a subject, the TI measurement signals being time-dependent and obtained during a time interval; generating, by the one or more processors, individually or in combination, using the TI measurement signals, respiration signals corresponding to the subject during the time interval; determining, by the one or more processors, individually or in combination, over the time interval, using the respiration signals, multiple values of a ratio of an exhalation time and an inhalation time of the subject; monitoring, by the one or more processors, individually or in combination, over the time interval, using the multiple values, a time-dependence of the ratio; and identifying, by the one or more processors, individually or in combination, based on the time-dependence, a status change of a cardiopulmonary condition of the subject. In some cases, the TI measurement signals may be received from a wearable device mounted on a torso of the subject.

In some aspects, the disclosure provides a computing system. The computing system includes: at least one processor; and at least one memory device storing processor-executable instructions that, in response to execution by the at least one processor, individually or in combination, cause the computing system at least to: receive thoracic impedance (TI) measurement signals corresponding to a subject, the TI measurement signals being time-dependent and obtained during a time interval; generate, using the TI measurement signals, respiration signals corresponding to the subject during the time interval; determine, over the time interval, using the respiration signals, multiple values of a ratio of an exhalation time and an inhalation time of the subject; monitor, over the time interval, using the multiple values, a time-dependence of the ratio; and identify, based on the time-dependence, a status change of a cardiopulmonary condition of the subject. In some cases, the TI measurement signals may be received from a wearable device mounted on a torso of the subject.

In some aspects, the disclosure provides a user device. The user device includes: at least one processor; and at least one memory device storing processor-executable instructions that, in response to execution by the at least one processor, individually or in combination, cause the user device at least to: receive thoracic impedance (TI) measurement signals corresponding to a subject, the TI measurement signals being time-dependent and obtained during a time interval; generate, using the TI measurement signals, respiration signals corresponding to the subject during the time interval; determine, over the time interval, using the respiration signals, multiple values of a ratio of an exhalation time and an inhalation time of the subject; monitor, over the time interval, using the multiple values, a time-dependence of the ratio; and identify, based on the time-dependence, a status change of a cardiopulmonary condition of the subject. In some cases, the TI measurement signals may be received from a wearable device mounted on a torso of the subject.

## BRIEF DESCRIPTION OF THE DRAWINGS

The accompanying drawings form part of the disclosure and are incorporated into the subject specification. The drawings illustrate example aspects of the disclosure and, in conjunction with the following detailed description, serve to explain at least in part various principles, features, or aspects of the disclosure. Some aspects of the disclosure are described more fully below with reference to the accompanying drawings. However, various aspects of the disclosure can be implemented in many different forms and should not be construed as limited to the implementations set forth herein. Like numbers refer to like elements throughout.

FIG. 1 is a schematic block diagram of an example of a system to monitor status changes of a cardiopulmonary condition, in accordance with one or more aspects of this disclosure.
FIG. 2A is a schematic diagram of an example of a user interface, in accordance with one or more aspects of this disclosure.

**FIG. 2B** presents plots of an example of volume of transacted air as a function of time during a breathing period and an example of thoracic impedance signal during the breathing period, in accordance with one or more aspects of this disclosure.

**FIG. 2C** presents a plot of an example volume of transacted air as a function of time during a time interval that includes a breathing period immediately followed by a pause in breathing, and a plot of an example of thoracic impedance signal during the time interval, in accordance with one or more aspects of this disclosure.

**FIG. 3** presents an example of a wearable device, in accordance with one or more aspects of this disclosure.

**FIG. 4** is a schematic diagram of a subject at two different instants during a pulmonary ventilation cycle, in accordance with one or more aspects of this disclosure.

**FIG. 5** is a plot of an example of the pulmonary ventilation signal obtained using a de-meaned thoracic impedance measurement signal of a subject, in accordance with one or more aspects of this disclosure.

**FIG. 6A** presents plots of inertial measurement signals as observed (or "raw") and after being processed in accordance with aspects of this disclosure, for two channels of a three-axis accelerometer.

**FIG. 6B** presents a plot of an example of a pulmonary ventilation signal and a plot of an example of a chest wall movement signal, in accordance with one or more aspects described herein.

**FIG. 7** presents plots of an example of thoracic impedance signal, an example of airflow signal, an example of chest wall movement signal, in accordance with one or more aspects described herein.

**FIG. 8A** presents plots of an example de-meaned thoracic impedance signal and an example of chest wall movement signal for a stable COPD, in accordance with one or more aspects described herein.

**FIG. 8B** presents plots of an example de-meaned thoracic impedance signal and an example of chest wall movement signal at peak exacerbation of COPD, in accordance with one or more aspects described herein.

**FIG. 9A** presents a schematic plot of a metric as a function of time, in accordance with one or more aspects of this disclosure.

**FIG. 9B** presents a schematic plot of two metrics as a function of time, in accordance with one or more aspects of this disclosure.

**FIG. 10** is deviation chart of SpO2 versus thoracic impedance for subjects having an acute exacerbation of chronic obstructive pulmonary disease (AECOPD), in accordance with one or more aspects of this disclosure.

**FIG. 11A** is a schematic block diagram of a processing platform to monitor status changes of a cardiopulmonary condition, in accordance with one or more aspects of this disclosure.

**FIG. 11B** is a schematic block diagram of an example of a computing device that can provide various functionality of monitoring status changes of a cardiopulmonary condition, in accordance with one or more aspects of this disclosure.

**FIG. 12** is a schematic block diagram of another example of a system to monitor status changes of a cardiopulmonary condition, in accordance with one or more aspects of this disclosure.

**FIG. 13** is a schematic block diagram of yet another example of a system to monitor status changes of a cardiopulmonary condition, in accordance with one or more aspects of this disclosure.

**FIGS. 14-17** illustrate example methods for determining a status change of a cardiopulmonary condition, in accordance with one or more aspects of this disclosure.

## DETAILED DESCRIPTION

Embodiments of the disclosure may addresses the issue of identification of changes in status of a cardiopulmonary condition of a subject. Cardiopulmonary conditions include, for example, chronic obstructive pulmonary disease (COPD) and congestive heart failure (CHF). As mentioned, COPD is a combination of emphysema and chronic bronchitis of various degrees of severity. Even when treated, these conditions can be progressively debilitating and can cause the subject to undergo significant medical intervention. Thus, monitoring the status of one or more cardiopulmonary conditions can help avoiding medical intervention. Monitoring the status of a cardiopulmonary condition typically involves ambulatory care. As a result, an exacerbation of the cardiopulmonary condition may be identified during a visit to a clinic or a clinician office. Thus, in some cases, the condition may have progressively deteriorated and/or may have been inadequately treated for a prolonged period of time prior to the visit to the clinic or the clinician office. Having a prolonged deterioration of the cardiopulmonary condition or inadequately treating the condition for a prolonged period of time can have dire consequences for the subject, which may in some cases result in costly medical interventions and unfavorable prognosis. Further, subjects may have difficulty adhering to a long-term routine of ambulatory care to monitor the status of cardiopulmonary conditions, which can lead to identification of a status change after a prolonged deterioration of the cardiopulmonary condition or when the subject is in distress. Both situations being clearly undesirable and detrimental to favorable prognosis.

Embodiments of technologies described herein provide systems, devices, techniques, and computer program products that, individually or in combination, permit monitoring a cardiopulmonary condition of a subject and/or contemporaneous identification of changes in the status of the cardiopulmonary condition. Such a monitoring is non-ambulatory, in that the monitoring can be performed without visiting a health services facility, and can be conducted in a dwelling (e.g., a home, a group home, a nursing home, etc.) that is remotely located from health services facilities. Thus, simply for the sake of nomenclature, the monitoring described herein can be referred to as non-ambulatory-care monitoring. Some systems in accordance with this disclosure include a wearable device that can be mounted on the torso of a subject. The wearable device can operate according to one or a combination of multiple sensing modalities, and can thus provide one or several types of measurement signals associated with bodily function of the subject. To that end, the wearable device may include multiple electrodes and multiple sensor devices. Each one of the multiple electrodes is configured to contact a respective skin area, at a respective skin location, on the torso of the subject. The multiple electrodes may be arranged in a particular layout on the chest of the subject, for example. The multiple sensor devices may include an electric sensor device functionally coupled with the multiple electrodes. The electric sensor device can measure a voltage, a current, an impedance, a combination thereof, or similar physical quantities. The electric sensor device is configured to generate measurement signals. The measurement signals may be generated during a measurement period. The measurement period can span several pulmonary ventilation cycles (also referred to as breathing cycles). The first measurement signals can include thoracic impedance (TI) measurement signals. The TI measurement signal can be generated in a four-point (or four-electrode) measurement configuration and at a defined sampling rate (e.g., 40 KHz).

In addition, or in some cases, the multiple sensor devices include one or more acoustic sensor devices configured to generate other measurement signals. Such other measurement signals may be generated during the measurement period. The acoustic sensor device(s), individually or in particular combination, can probe sounds originating in or propagating through an organ that is present within the chest cavity and/or adjacent areas. For example, one or more first acoustic sensor devices of the acoustic sensor device(s) can serve as lung auscultation probe(s).

Further, or in yet other cases, the multiple sensors include an inertial sensor device configured to generate yet other measurement signals. Such other measurements signals may be generated during the measurement period. The inertial sensor device can be a three-axis accelerometer, and the measurement signals generated by the inertial sensor device can be used to generate movement signals corresponding to movement of the chest wall of the subject during a time interval, such as the measurement period.

Further, or in some configurations, the multiple sensor devices also include an optical device that permits measuring oxygen saturation level, and can generate measurement signals accordingly. For example, the optical device can be a pulse oximeter. In addition, or in other configurations, the multiple sensor devices also could include a blood pressure meter.

It is noted that embodiments of the technologies described herein are not limited to the sensor devices described herein. More sensor devices or fewer sensor devices may form part of a wearable device in accordance with aspects of this disclosure.

Systems in accordance with aspects of this disclosure also can include a processing platform having multiple computing devices. Computing devices can be server devices arranged in a cloud architecture. At least one of the server devices can operate as a gateway device. At least one of the server devices can operate as a compute node. At least one of the server devices can operate as storage node.

The processing platform may be functionally coupled or otherwise configured to communicate with the wearable device. In some cases, the processing platform is functionally coupled with the wearable device via one or more networks (wireless network(s) or wireline network(s), or a combination of both). The processing platform, via the network(s) and at least one of the multiple computing devices, can receive measurement signals generated by the wearable device over time. The processing platform, via at least one of the multiple computing devices, can implement numerous processes based on the received measurement signals. At least some of the processes can include determining values of a particular metric or a combination of metrics based on the measurement signals. In some cases, the particular metric is indicative of status of a cardiopulmonary condition of a subject. In addition, or in other cases, the combination of metrics is indicative of the status of the cardiopulmonary condition of the subject. Examples of the metrics include respiratory rate (RR), tidal volume (TV), ratio of an exhalation time and an inhalation time of the subject, delay between mechanical onset of inspiration and onset of inspiration airflow. It is noted that a metric can be a quantity directly probed by measurement signals generated by a sensor device. For instance, a metric can be blood oxygen level directly measured by an oximeter present in the wearable device.

At least some of the processes, when implemented, can permit tracking temporal changes of multiple metrics. Thus, the process(es) can include determining multiple values of a particular metric or a combination of metrics over a time interval. The time interval spans two or more measurement intervals, each of the measurement intervals result in a value of a metric for each one of the metrics being tracked. The process(es) also can include monitoring, over the time interval, using the multiple values, a time-dependence of the particular metric or the combination of metrics. In some cases, monitoring such a time-dependence can include updating parameters defining a function (linear or otherwise) that fits currently determined values of the particular metric or the combination of metrics. In addition, or in other cases, monitoring such a time-dependence can include updating a parameter defining a rolling average of currently determined values of the particular metric or the combination of metrics. Regardless of how the time-dependence is monitored, the time-dependence of either a single metric or a combination or metrics can convey a trend (e.g., an upward trend, a downward trend, or an essentially constant trend). Thus, by monitoring one or more metrics individually and/or combinations of two or more metrics, embodiments of the technologies described herein can provide detection and differentiation of cardiopulmonary exacerbations. As such, embodiments of the technologies described herein provide an improvement over commonplace technologies for the monitoring and management of cardiopulmonary conditions.

At least some of the systems in accordance with the technologies described herein also include a computing device that can be remotely located relative to the processing platform. The computing device also can be functionally coupled or otherwise configured to communicate with the processing platform via one or more networks. In some cases, such network(s) can be the same network(s) that permit functionally coupling the wearable device with the processing platform described herein. The computing device can be associated with, or otherwise operated by, a clinician and/or an insurance provider. The clinician can be involved in providing care for cardiopulmonary condition of the subject. As such, the computing device can receive information related to a status of the cardiopulmonary condition of the subject and/or a change in the status of the pulmonary condition of the subject. At least a portion of the information can be formatted as an electronic message that can be presented by a software application, or another type of program code, configured to execute in the computing device. The computing device, via the software application, can receive and present the information. The software application may be a mobile application, a web browser, or another type of executable program code.

The computing device also can be functionally coupled or otherwise configured to communicate to a user device via one or more networks. In some cases, such network(s) can be the same network(s) that permit functionally coupling the computing device with the processing platform described herein. In some configurations, the user device can be part of one or more of the systems in accordance with the technologies described herein. The user device can be associated with, and operated by, the subject wearing the wearable device and/or by an aid (e.g., a family member or another type of caregiver, such as a nurse, a nurse assistant, or nursing home staff) assisting the subject with performing measurements. The user device is configured with a software application, or another type of program code, to exchange information with the computing device. As such, the computing device, via the software application configured in the computing device, can provide an indication of an action to be executed by the subject in response to a particular value of a metric monitored being monitored and/or a status change in the cardiopulmonary condition. The type of action can be associated with the nature of the status change. The indications of actions that a subject may perform or cause to perform may be provided as the subject monitors a cardiopulmonary condition of the subject over a measurement period, a test period, and/or an analysis period, in accordance with aspects described herein. Accordingly, various actions directed to improving the monitoring of the cardiopulmonary condition and/or improving the cardiopulmonary condition itself, can be implemented with much greater immediacy than in commonplace technologies and approaches for monitoring the cardiopulmonary condition. As a result, embodiments of the technologies described herein provide an improvement over commonplace technologies and approaches for monitoring and/or managing cardiopulmonary conditions.

The user device mentioned above can be functionally coupled or otherwise configured to communicate with the wearable device. The user device also can be functionally coupled or otherwise configured to communicate with the processing platform via one or more networks. In some cases, such network(s) can be the same network(s) that permit functionally coupling the wearable device with the processing platform described herein.

The software application, or another type of program code, that is configured in the user device can control the manner of performing measurements using the wearable device. To that end, in response to the software application being executed, the user device can present a user interface that permits configuring a measurement session, e.g., a measurement period, number of measurements to be performed during the measurement period, a combination thereof, or similar attributes of the measurement session. The user device also can present one or more other user interfaces that display various types of information, including observed data (e.g., electrocardiogram (ECG) data, thoracic impedance, SpO2, etc.) from measurements.

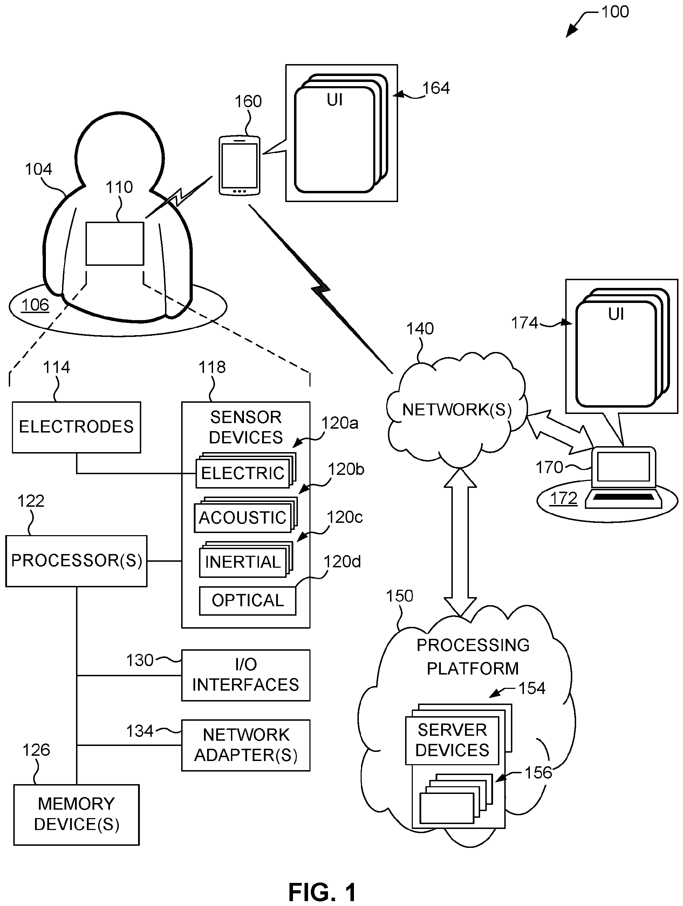

**FIG. 1** is a schematic block diagram of an example of a system 100 to monitor status changes of a cardiopulmonary condition in accordance with aspects of this disclosure. The example system 100 can include a wearable device 110 that can be mounted on the torso of a subject 104. Although the wearable device 110 is depicted as mounted on a particular side of the torso of the subject 104, placement of the wearable device 110 is not limited in that respect. In some cases, when mounted on the subject 104, the wearable device 110 can extend from one side of the torso to another side of the torso. As such, the wearable device 110 can be mounted on the anterior side of the torso, on the posterior side of torso, on a lateral side of torso, or in an arrangement that extends from one side (e.g., anterior, posterior, or lateral) to another different side of the torso. The wearable device 110 can operate according to one or a combination of multiple sensing modalities. The wearable device 110 can thus provide one or several types of measurement signals associated, individually or in combination, with a bodily function of the subject 104. The bodily function can be respiration or cardiac function, for example. The wearable device 110 includes multiple electrodes 114 and multiple sensor devices 118. Each one of the multiple electrodes 114 is configured to contact a respective skin section of the torso of the subject 104.

The multiple sensor devices 118 include a group of electric sensor devices 120a coupled with the multiple electrodes 114. Each electric sensor device of the group of electric sensor devices 120a can measure a voltage, a current, an impedance, a combination thereof, or similar physical quantities. In addition, each electric sensor device of the group of electric sensor devices 120a is configured to generate a measurement signal during a measurement period. The measurement signal can be referred to as electrical measurement signal. Electric measurement signals generated over time can be used to monitor a cardiopulmonary condition by probing pulmonary ventilation characteristics and/or cardiac characteristics of the subject 104. Hence, as is described herein, the measurement period can span several pulmonary ventilation cycles (also referred to as breathing cycles), each cycle extending between initiation of inhalation and termination of exhalation. A measurement period may have a short duration corresponding to the time spanned by a few or several breaths of the subject. In addition, or in other cases, a measurement period may have a long duration that may range from a few minutes to a few days. Simply as an illustration, in some cases, the measurement period has a duration within a range from about 15 seconds to about 120 seconds. Examples of the measurement period include 15 s, 25 s, 30, s, 45 s, 60 s, 70 s, 80 s, 90 s, 100 s, 110 s, and 120 s. As another illustration, in other cases, the measurement period has a duration within a range from about 25 seconds to about five days.

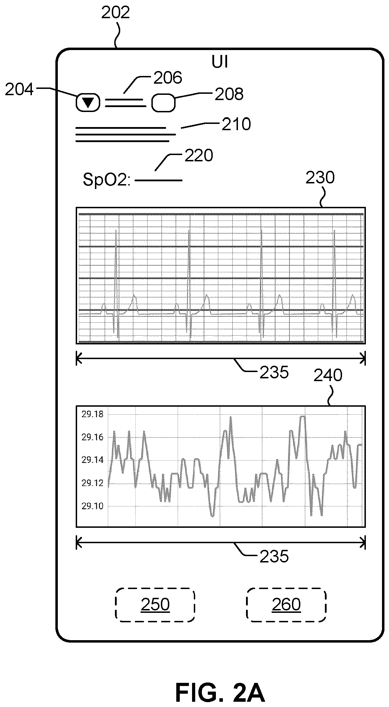

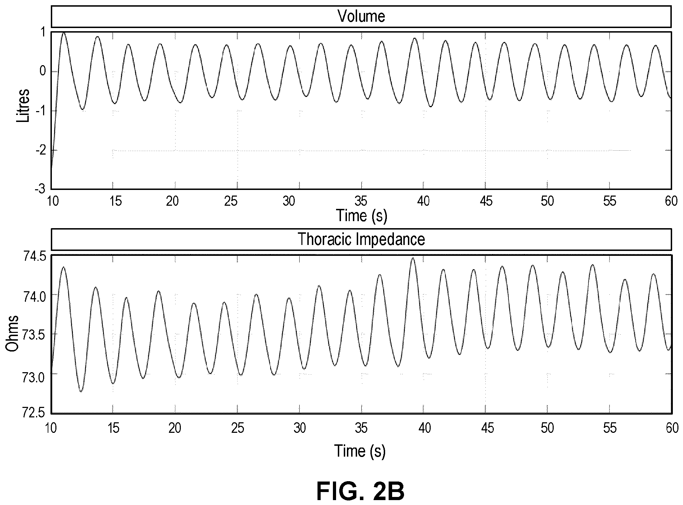

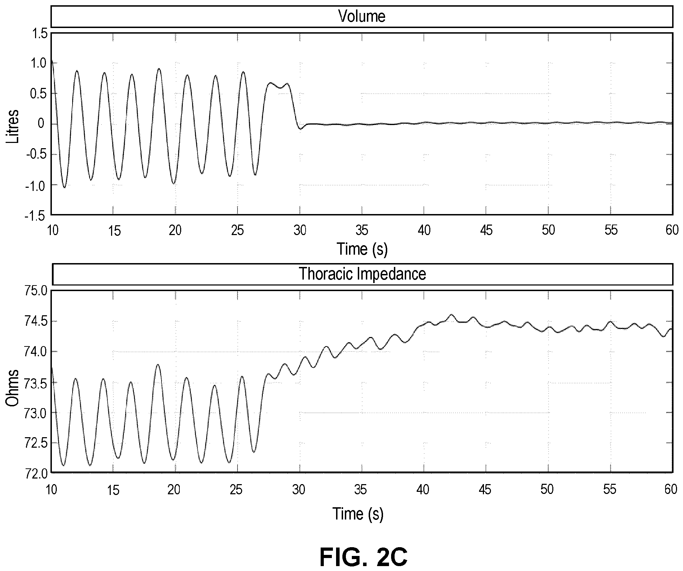

A particular electric sensor device of the group of electric sensor devices 120a measures impedance. Thus, the particular electric sensor device is configured to generate measurement signals indicative or otherwise representative of impedance. Because the wearable device 110 is mounted on the torso of the subject 104 (e.g., on the chest of the subject 104), the impedance that the particular electric sensor measures is thoracic impedance (TI). Accordingly, the measurement signals can be referred to as thoracic impedance (TI) measurement signals. The TI measurement signals are generated in a four-point configuration (or four-electrode measurement), in some cases. As is illustrated in the examples shown in **FIG. 2B** and **FIG. 2C**, a TI measurement signal generated over a measurement period is representative of amount of air that is present in the lungs as the subject 104 breaths during the measurement period. That is, the temporal dependence of TI measurement signal represents the amount of air present in the lungs as a function of time. Accordingly, measurements of thoracic impedance that are performed by the wearable device 110 can substitute measurements of the amount of air that is present in the lungs during breathing.

Because measurements of thoracic impedance can be performed where the subject 104 is located, such measurements permit monitoring pulmonary ventilation characteristic of the subject 104 without reliance on a health services facility to access dedicated equipment (such as spirometry equipment) and be subjected to measurements of the volume of air that enters and exits the lungs of the subject 104. As a result, in sharp contrast to conventional technologies and ambulatory-care monitoring, the monitoring in accordance with aspects of this disclosure provides a wealth of pulmonary ventilation data with a time resolution that is superior to that of commonplace ambulatory-care monitoring.

Other particular electric sensor device of the group of electric sensor devices 120a can generate ECG data. To that end, the particular electric sensor device can measure voltages or voltage differences corresponding to respective particular electrodes or combination of electrodes of the multiple electrodes 114. The particular electrodes, individually or in combination, permit measuring voltages corresponding to leads that define an ECG vector. For instance, particular combinations of electrodes can configure respective leads, each of which leads defines an ECG vector. Those leads may correspond, or, in some cases, may be similar, to at least a subset of standard precordial leads (V1, V2, V3, V4, V5, V6) and standard limb leads (I, II, III, aVF, aVL, and aVR). Thus, the number of particular electrodes permits configuring at least three leads used either (i) to generate ECG data defining a 12-lead ECG or (ii) to permit reconstructing the 12-lead ECG based on machine-learning models or other techniques.

Back to referring to **FIG. 1,** the multiple sensor devices 118 (**FIG. 1**) also include a group of acoustic sensor devices 120b. Each acoustic sensor device of the group of acoustic sensor devices 120b is configured to generate measurement signals. The measurement signals may be generated during a measurement period. The group of acoustic sensor devices 120b, individually or in a particular combination, can probe sounds originating in or propagating through an organ that is present within the chest cavity of the subject 104. In one example, the organ is a lung and the one or more first acoustic sensor devices of the acoustic sensor device(s) 120b can serve as respective lung auscultation probes. As such, the first acoustic sensor device(s) can be mounted on the torso of the subject (e.g., chest of the subject) according to various configurations. In one configuration, the first acoustic sensor device(s) can be distributed on a same section of the torso of the subject 104. Such a section can be an anterior section, a posterior section, or a lateral section, for example. In another configuration, the first acoustic sensor device(s) can be distributed on different sections of the torso of the subject 104. As an illustration of such a configuration, a first acoustic sensor device can be placed on an anterior section of the torso of the subject 104, and a second acoustic sensor device can be placed on a lateral section of the torso of the subject 104. In such a configuration, the first and second sensor can probe sound propagation within a lung of the subject 104. Specifically, the time elapsed during sound propagation between the first and second acoustic sensor can be measured. Such a time can be indicative or otherwise representative of an amount of fluid within the lung.

The multiple sensor devices 118 further include a group of inertial sensor devices 120c. The wearable device 110 is mounted on the torso of the subject 104 such that each inertial sensor device is mechanically coupled to the chest of the subject 104. Specifically, each inertial sensor can be in contact with a solid medium that, in turn, is in contact with the chest of the subject 104. In some cases, the group of inertial sensor devices 120c consists of a single inertial sensor device. As an example, the single inertial sensor device can include a three-axis accelerometer. In other cases, the group of inertial sensor devices 120c includes multiple inertial sensor devices, e.g., a three-axis accelerometer and a three-axis gyroscope.

Regardless of type, number, and arrangement of the inertial sensor devices, each inertial sensor device of the group of inertial sensor devices 120c is configured to generate measurement signals. The measurement signals may be generated during a measurement period. The measurement signals are generated as the subject 104 breaths, and can be referred to as inertial measurement signals (or acceleration measurement signals). An inertial measurement signal can be used to generate a movement signal indicative or otherwise representative of movement of the chest wall of the subject 104 during a measurement period. Over a time interval including two or more test periods, inertial measurement signals can thus be used to monitor the movement of the chest wall of the subject 104 as the subject breaths, over the time interval.

It is noted that in some implementations, the multiple sensor devices 118 may include other types of movement sensor devices instead of inertial sensor device(s). A movement sensor device may be, or may include, a transducer device that converts kinetic energy corresponding to movement of the chest wall of the subject 104 to an output signal (e.g., an electric signal, a magnetic signal, an electromagnetic signal, and/or a delay signal) indicative or otherwise representative of the movement of chest wall of the subject 104. The output signal may thus be referred to as a movement signal. As an example, such a movement sensor device may include a piezoelectric material that can transduce an amount of displacement of the chest wall into an electric signal (e.g., a voltage signal or a current signal), with the electric signal being indicative of otherwise representative of the movement of the chest wall of the subject 104. As another example, a movement sensor device may be, or may include, an optoelectronic assembly having light source device(s) and photodetector device(s) that can measure an amplitude, direction, and/or velocity of movement of the chest wall. The optoelectronic assembly can be configured to generate one or more electric signals (e.g., current signals) and, in some cases, delay signal(s) that, individually or in combination, indicate or otherwise represent the movement of the chest wall of the subject 104.

With further reference to **FIG. 1,** in cases where a first inertial sensor device of the group of inertial sensor devices 120c is a three-axis accelerometer, the three-axis accelerometer generates three inertial measurement signals corresponding to three orthogonal axes, such as the axes $x$, $y$, and $z$ of a Cartesian coordinate system. The $z$ axis can be oriented along the direction of gravity. The inertial measurements can collectively represent (i) an orientation of the three-axis accelerometer (or the wearable device 110) relative to the chest wall of the subject 104 and (ii) a rectilinear acceleration vector. Without intending to be bound by modeling, during regular breathing the magnitude of the acceleration vector is negligibly small relative to the acceleration of gravity because changes in the second derivative with respect to time of a displacement vector of the chest wall is negligibly small. For example, the magnitude of the acceleration vector can be approximately three orders of magnitude less than the acceleration of gravity. Hence, the inertial measurement signal can essentially be deemed representative of the orientation of the three-axis accelerometer relative to the chest wall of the subject 104. In a scenario where the subject 104 lays on their back during a measurement period, the orientation of the three-axis accelerometer relative to the chest wall of the subject 104 is defined by a tilt angle relative to a horizontal plane that supports the subject 104. Accordingly, the three-axis accelerometer is in practice a tilt sensor device.

In terms of a Cartesian coordinate system, the three inertial measurement signals are indicative, respectively, of a first component $a_x$ (along the x axis) of the gravity acceleration vector; a second component $a_y$ (along the $y$ axis) of the gravity acceleration vector; and a third component $a_z$ (along the z axis) of the gravity acceleration vector. The first component and second component define an in-plane component $a_h = a_x \cos(\varphi) + a_y \sin(\varphi)$, where $\varphi$ is the polar angle coordinate of $a_h$ in the $(x,y)$ plane of the three-axis accelerometer (or the wearable device 110); that is, $\varphi$ is the angle of rotation of $a_h$ about the z axis.

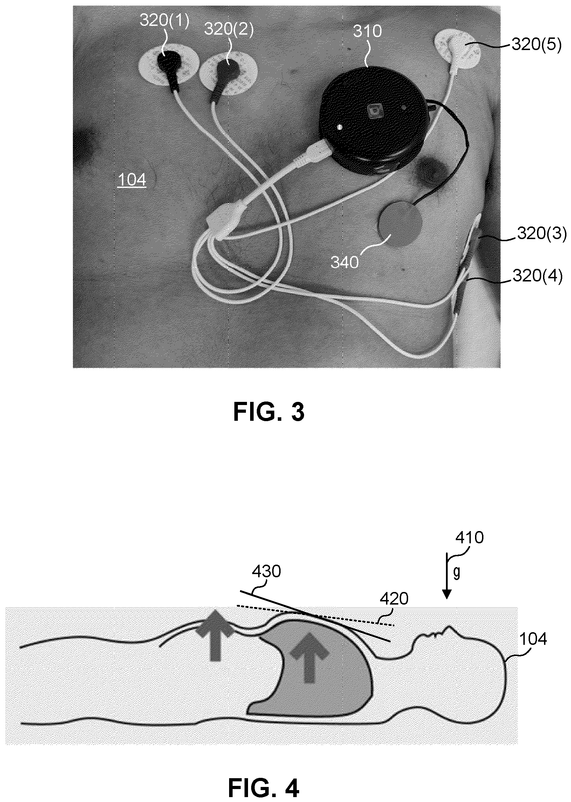

As the subject breaths, the position of the three-axis accelerometer (or the wearable device 110) changes along an axis 410 that is parallel to the direction of gravity. A change $\Delta h$ of such a position relative to a position of the three-axis accelerometer (or the wearable device 110) at the end of exhalation is proportional to a change $\Delta a_h$ of the in-plane component $a_h$. In **FIG. 4,** the $(x,y)$ plane at the end of exhalation is represented by a dotted segment 420, and the $(x,y)$ plane at the peak in inhalation is represented by a solid segment 430. The change in orientation of the three-axis accelerometer due to movement of the chest wall during a breath cycle is mainly represented by the change in the components of the gravity acceleration vector on the $x$, $y$, and $z$ axes. In particular, without intending to be bound by theory and/or modeling, the change in the in-plane component $a_h$ can be modeled as $\Delta a_h = \Delta a_x \cos(\varphi) + \Delta a_y \sin(\varphi)$, where $\Delta a_x$ and $\Delta a_y$ are the variation in the first component $a_x$ and the variation of the second component $a_y$ as measured by the three-axes accelerometer, respectively. Therefore, movement of the chest wall of the subject 104 can be monitored by determining $\varphi$, and processing a first inertial signal indicative of $\Delta a_x$ and a second inertial signal indicative of $\Delta a_y$.

A value of $\varphi$ is determined for each measurement period because the wearable device 110 may be removed after a prior measurement period and replaced in a current measurement period. In some cases, a satisfactory (e.g., best, or second-best) value of $\varphi$ can be determined iteratively by maximizing the correlation between $\Delta a_h$ and the pulmonary ventilation signal obtained from a thoracic impedance signal. In some cases, the correlation can be a shape based correlation. Thus, to calculate such a correlation (denoted by $\rho$), for a first data series $\{u\}$ and a second data series $\{v\}$, the cross-covariance of $\{u\}$ and $\{v\}$ (denoted by $cov(u,v)$) can be determined. Then, the cross-covariance of $\{u\}$ and $\{v\}$ can be normalized with respect to the standard deviations of $\{u\}$ and $\{v\}$. Namely, $\rho = cov(u, v)/(\sigma_u \sigma_v)$, where $\sigma_u$ and $\sigma_u$ represent, respectively, the standard deviation of $\{u\}$ and $\{v\}$. In the iterative determination of $\varphi$, $\{u\}$ is a time series of values of $a_x$ and $\{v\}$ is a time series of values of $a_y$.

In some configurations, the multiple sensor devices 118 also include an oximeter device 120d that can permit measuring oxygen saturation level. The oximeter device 120d is configured to generate measurement signals during a measurement period. In addition, or in other configurations, the multiple sensor devices 118 also include a blood pressure meter (not shown in **FIG. 1**).

It is noted that embodiments of the technologies described herein are not limited to the multiple sensor devices 118 described herein. Further, an electric sensor device of the group of electric sensor devices 120a can be used to measure temperature of the subject 104.

The wearable device 110 is depicted as a single block mounted on the torso of the subject 104 simply for purposes of illustration. Although the wearable device 110 is monolithic in some implementations, there are other implementations in which the wearable device 110 is not monolithic. **FIG. 3** presents an example of an arrangement of the multiple electrodes 114 and a housing 310 that holds the multiple sensor devices 118, the processor(s) 122, the memory device(s) 126, the input/output (I/O) interfaces 130, the network adapter(s) 134, and, in some cases, other components. In **FIG. 3**, the multiple electrodes 114 include four electrodes 320(1), 320(2), 320(3), and 320(4) used for four-point thoracic impedance measurements. In addition, the wearable device 110 that is illustrated in **FIG. 3** also includes a fifth electrode 320(5) that embodies the left arm (LA) used in multiple leads for electrocardiogram measurements. The fifth electrode 320(5) can serve as a reference electrode. The wearable device 110 that is illustrated in **FIG. 3** also includes a piezoelectric transducer 340 that is part of an acoustic sensor (not shown in **FIG. 3**) and permits measuring heart sounds. In some cases, the piezoelectric transducer 340 may be used in combination with another piezoelectric transducer (not shown) to probe sound propagation across a lung of the subject 104. Electrodes 320(1), 320(2), 320(3), 320(4), and 320(5), and the piezoelectric transducer 340 are connected with the multiple sensor devices 118 (not shown in **FIG. 3**) via cables and a single connector/adapter at the housing 310.

The wearable device 110 also includes one or more processors 122, one or more memory devices 126, multiple I/O interfaces 130, and one or more network adapters 134. At least one of the processor(s) 122 and at least one of the memory device(s) 126 can form a control unit. In some cases, the control unit can implement control operations involved in setup/placement of the wearable device 110 and/or performance of one or more measurements by at least one of the multiple sensor devices 118, resulting in measurement signals. A first network adapter of the network adapter(s) 134 can be embodied in a radio unit that permits exchanging information wirelessly between the wearable device 110 and another device.

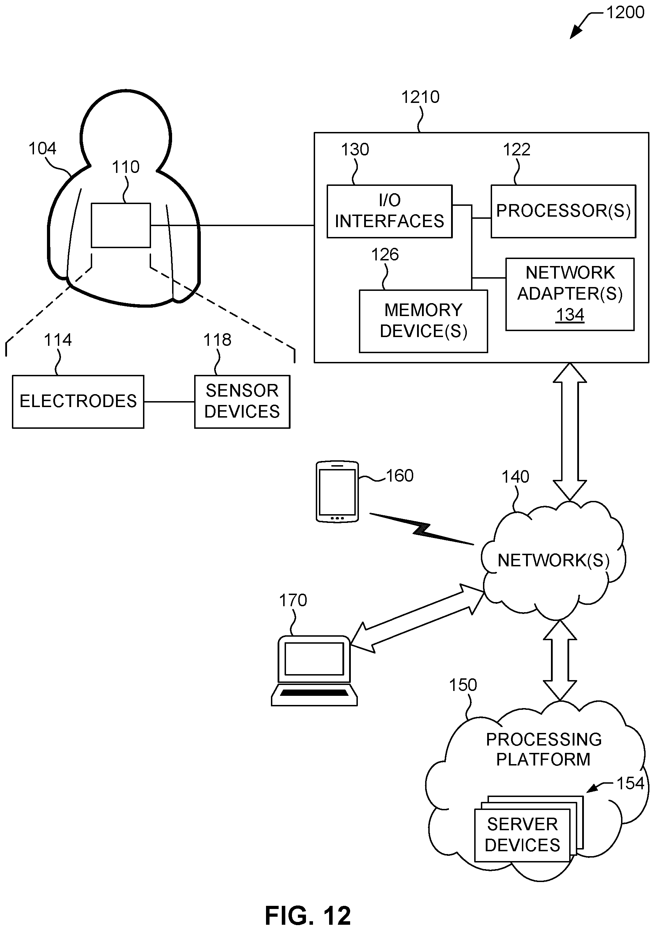

The wearable device 110 can be functionally coupled or otherwise configured to communicate with a processing platform 150 that also is part of the example system 100. Such functional coupling can be implemented in numerous ways, and can permit the wearable device 110 to supply data and/or signaling (control instructions, for example) to the processing platform 150. In some cases, as is illustrated in **FIG. 1**, the wearable device 110 is functionally coupled or otherwise configured to communicate with the processing platform 150 via a user device 160 and one or more networks 140 (wireless network(s) or wireline network(s), or a combination of both). In other cases (as is shown in **FIG. 12**) the wearable device 110 is functionally coupled or otherwise configured to communicate with the processing platform 150 via the network(s) 140, without the user device 160 serving an intermediary element that permits such coupling.

The user device 160 is depicted as a smartphone simply for the sake of illustration. This disclosure is not limited in that respect. Indeed, the user device 160 can be any device having computing resources that, individually or in combination, can execute processor-accessible instructions and can cause a display device to display information. The processor-accessible instructions may be processor-executable program code. In some cases, the processor-executable program code may be embodied in software libraries, application programming interfaces (APIs), processor-executable modules, and the like. The display device may be integrated into the user device 160 or functionally coupled therewith. Further, the user device 160 may be mobile (handheld or otherwise) or pseudo-stationary. Besides a smartphone, examples of the user device 160 include a phablet device, a tablet computer, a laptop computer, or similar apparatuses.

The processing platform 150 includes multiple server devices 154. The processing platform 150, via the network(s) 140 and at least one first server device of the multiple server devices 154, can receive measurement signals generated by the wearable device 110 over time. The processing platform 150, via at least one second server device of the multiple server devices 154, can implement numerous processes that use the received measurement signals. Such processes can permit determining status or status change of a cardiopulmonary condition of the subject 104. The at least one second server device can retain a group of modules 156 that, in response to being executed by processor(s), cause the at least one second server, individually or in combination, to perform at least one of the processes. More specifically, at least some of such processes can include determining values of a particular metric or a combination of metrics based on the measurement signals. In some cases, the particular metric is indicative of status of a cardiopulmonary condition of the subject 104. In addition, or in other cases, the combination of metrics is indicative of the status of the cardiopulmonary condition of the subject 104.

The processing platform 150 can evaluate various types of metrics that can permit, individually or in combination, identifying a status change of a cardiopulmonary condition. In this disclosure, a type of a metric can be direct type or aggregate type. A metric of the direct type (referred to as a direct metric) is a quantity that is directly probed by a sensor device of the multiple sensor devices 118. That is, measurement signals generated by the sensor device are indicative of respective values of the metric. The quantity can be a physical quantity, a chemical quantity, or a physicochemical quantity. Examples of the physical quantity include thoracic impedance and speed of sound propagation. An example of the chemical quantity is oxygen saturation. As mentioned, the oximeter device 120d can directly probe oxygen saturation level. Thus, a measurement signal generated by the oximeter device 120d is indicative of the oxygen saturation level. The second server device(s) can then obtain an observed value indicating the oxygen saturation level from the measurement signal generated by the oximeter device 120d. The second server device(s) can then assign the observed value to a direct metric corresponding to oxygen saturation.

A metric of the aggregate type (referred to as aggregate metric) is a quantity derived by operating on measurement signals generated by a sensor device of the multiple sensor devices 118. The second server device(s) can operate on measurements signals received from the wearable device 110. The second server device(s) operate on measurement signals according to one or more processes corresponding to the aggregate metric being evaluated. Some aggregate metrics can be derived by operating on one type of measurement signals, e.g., TI measurement signals. Other aggregate metrics can be derived by operating on two or more types of measurement signals. Examples of aggregate metrics that can be derived by operating on TI measurement signals include respiratory rate (RR), tidal volume (TV), and ratio of exhalation time and inhalation time. Such a ratio is herein denoted by $\rho_{EI}$, simply for the sake of nomenclature. Examples of aggregate metrics that can be derived by operating on TI measurement signals and inertial measurement signals include delay between mechanical onset of inspiration and onset of inspiration airflow.

To determine a value of an aggregate metric associated with pulmonary ventilation, a server device of the multiple server devices 154 can generate a pulmonary ventilation signal (also referred to as respiration signal) by processing a TI measurement signal, and can then evaluate the aggregate metric using the pulmonary ventilation signal. Processing the TI measurement signal includes filtering the TI measurement signal using a low-pass filter configured to remove frequencies exceeding a cutoff frequency within a breathing bandwidth. Here, the breathing bandwidth is a range of frequencies that contains essentially all likely frequencies of pulmonary ventilation in an adult human. The range of frequencies can extend, for example, from about 0.05 Hz to about 0.75 Hz. In one example, the cutoff frequency is 0.75 Hz. By filtering the TI measurement signal in such a fashion, frequency components associated with other bodily functions besides breathing can be removed from the TI measurement signals. The processing also includes subtracting a mean value of the filtered TI measurement signal from the TI measurement signal. Subtracting such a mean value yields a de-meaned TI measurement signal. The processing further includes subtracting a moving average of the de-meaned TI measurement signal from the de-meaned TI measurement signal. Subtracting such a moving average can remove baseline drift within a test period corresponding to the TI measurement signal, and results in the pulmonary ventilation signal.

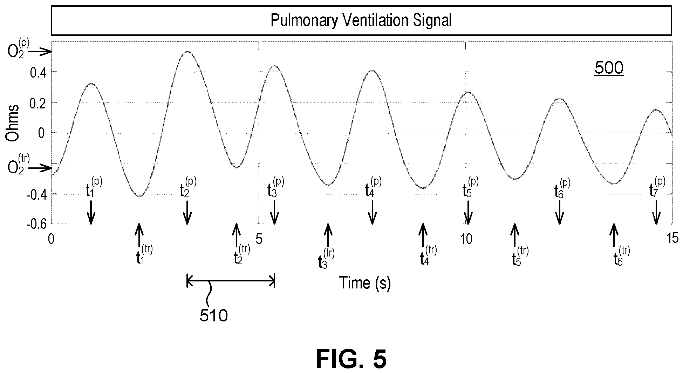

A plot 500 of an example of the pulmonary ventilation signal is shown in the **FIG. 5.** The pulmonary ventilation signal spans 15 seconds, which can be simply an example of a measurement period. The pulmonary ventilation signal is cyclic and, thus, includes various maxima (or peaks) and minima (or troughs). The pulmonary ventilation signal permits determining various respiratory parameters. Specifically, respiratory cycle duration can be determined as mean of time differences $\Delta t^{(i)} = t_{k+1}^{(p)} - t_k^{(p)}$ between successive peaks and/or time differences $\Delta t^{(k)} = t_{k+1}^{(tr)} - t_k^{(tr)}$ between successive troughs. In **FIG. 5,** the index $k$ denotes a cycle and adopts the values 1, 2, 3, 4, 5, and 6. In addition, tidal volume can be determined as the difference between peak impedance $O_k^{(p)}$ and trough impedance $O_k^{(tr)}$ for a cycle $k$ (1, 2, 3, 4, 5, or 6). For example, for cycle 510, tidal volume can be determined as $O_2^{(p)} - O_2^{(tr)}$. Further, inhalation time can be determined as the difference $T_i^{(k)} = t_k^{(p)} - t_{k-1}^{(tr)}$ between time of a peak in a cycle and time of a trough in an immediately preceding cycle. Furthermore, exhalation time can be determined as the difference $T_e^{(k)} = t_k^{(tr)} - t_{k-1}^{(p)}$ between time of a trough in a cycle and time of a peak in an immediately preceding cycle.

Evaluating the aggregate metric using the pulmonary ventilation signal includes identifying maxima (or peaks) and minima (or troughs) of the pulmonary ventilation signal. To that end, a peak and trough detection process can be

10

applied to the pulmonary ventilation signal. Such a detection process can include determining multiple extrema of the pulmonary ventilation signal by applying a time-domain zero-crossing process to a first order derivative with respect to time of the pulmonary ventilation signal. The detection process also can include determining multiple peaks within the extrema of the pulmonary ventilation signal by, at least in part, determining a second order derivative with respect to time of the pulmonary ventilation signal. The multiple peaks have respective first zero-crossing times, and at such times, the second order derivative with respect to time of the pulmonary ventilation signal is negative. The detection process can further include determining multiple troughs within the extrema of the pulmonary ventilation signal by, at least in part, determining the second order derivative with respect to time of the pulmonary ventilation signal. The multiple troughs have respective second zero-crossing times, and at such times, the second order derivative with respect to time of the pulmonary ventilation signal is positive.

Evaluating the aggregate metric also includes determining, using the maxima and/or minima of the pulmonary ventilation signal, a series of values ($\mu_n$) of the aggregate metric over the test period corresponding to the pulmonary ventilation signal. The definition of the aggregate metric dictates whether the series of values is determined using maxima alone, minima alone, or a combination of maxima and minima. Additionally, evaluating the aggregate metric also includes determining a representative value $\bar{\mu}$ of the series of values $\{\mu_n\}$, and configuring $\bar{\mu}$ as the value of the aggregate metric for the test period corresponding to the pulmonary ventilation signal. The representative value $\bar{\mu}$ may be a median, mean, the modal value, or some other statistically significant representative value of the series of values $\{\mu_n\}$.

As mentioned, some aggregate metrics can be evaluated using two or more types of measurement signal. For example, delay between mechanical onset of inspiration and onset of inspiration airflow is an aggregate metric that is evaluated by operating on TI measurement signals and inertial measurement signals. Such a delay represents a relationship between mechanical breath and air flow and, depending on magnitude, can be indicative of presence or absence of certain cardiopulmonary conditions.

To determine a delay between mechanical onset of inspiration and onset of inspiration airflow, a server device of the multiple server devices 154 can generate a movement signal representative of movement of the chest wall of the subject 104 based on an inertial measurement signal and a TI measurement signal during a measurement period. Movement of the chest wall can be represented by a linear combination (i) a first inertial measurement signal representative of a first component of gravity along a first direction and (ii) a second inertial measurement signal representative of a second component of gravity along a second direction. The first direction and second direction are contained within a plane of the wearable device 110. The first inertial measurement signal and the second inertial measurement signal can be generated, respectively, by a first channel and a second channel of a three-axis accelerometer. Specifically, the first inertial measurement signal and the second inertial measurement signal can be representative of $\Delta a_x$ and $\Delta a_y$, respectively. Thus, the movement of the chest wall can be represented by $\Delta a_x \cos(\varphi) + \Delta a_y \sin(\varphi)$.

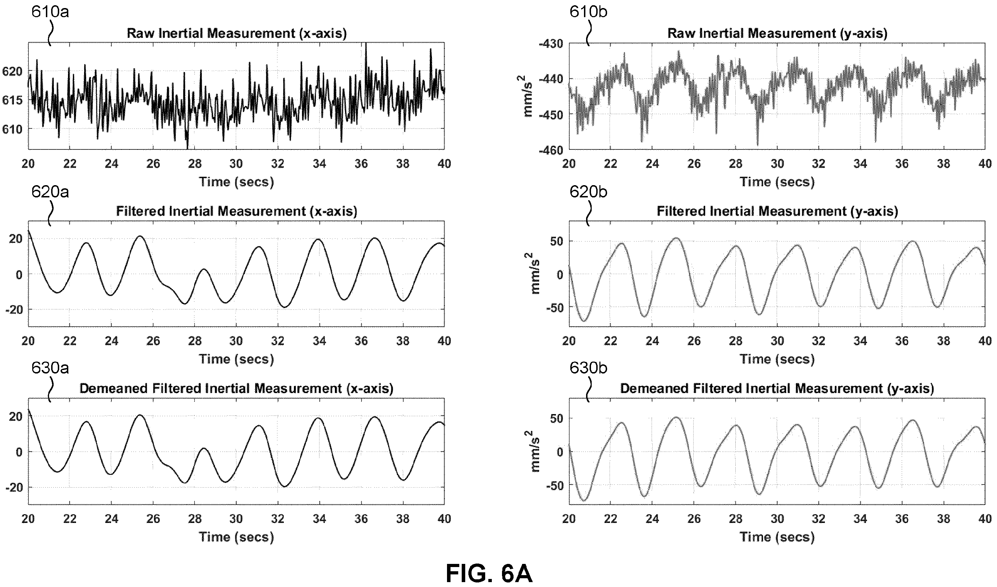

Hence, to generate such a movement signal, the server device can process the first inertial measurement signal (representing $\Delta a_x$) and the second inertial measurement signal (representing $\Delta a_y$). In **FIG. 6A,** a plot 610a and a plot 610b present examples of the first inertial measurement signal and the second inertial measurement, respectively. Processing such inertial measurement signals includes filtering that first inertial measurement signal using a low-pass filter configured to remove frequencies exceeding a cutoff frequency within a breathing bandwidth. Here, as mentioned, the breathing bandwidth can span a range of frequencies that extends, for example, from about 0.05 Hz to about 0.75 Hz. In one example, the cutoff frequency is 0.75 Hz. The processing also includes filtering that second inertial measurement signal using the low-pass filter. In **FIG. 6A,** a plot 620a and a plot 620b present examples of the filtered first inertial measurement signal and the filtered second inertial measurement, respectively. The processing also includes subtracting a mean value of the filtered first inertial measurement signal from the filtered first inertial measurement signal. Subtracting such a mean value yields a de-meaned first inertial measurement signal. The filtered second inertial measurement signal also is de-meaned. That is, the processing further incudes subtracting a mean value of the filtered second inertial measurement signal from the filtered second inertial measurement signal. Subtracting such a mean value yields a de-meaned second inertial measurement signal. In **FIG. 6A,** a plot 630a and a plot 630b present examples of the de-meaned first inertial measurement signal and the de-meaned second inertial measurement, respectively.

The processing also involves the TI measurement signal. Specifically, the processing includes converting each one of first de-meaned inertial measurement signal and the second de-meaned inertial measurement signal to the time base of the TI measurement signal. Such a conversion permits directly comparing those inertial measurement signals and the TI measurement signal (or a signal derived therefrom) in the same time base. To perform such a conversion, a time grid can be generated from the time base of the TI measurement signal, using a start time and an end time of the TI measurement signal, and also using a sampling duration of the TI measurement signal. The sampling duration is obtained from the sampling rate of the measured TI. Additionally, each one of the first and second de-meaned inertial measurement signal is then interpolated at the times in the time grid, thus providing de-meaned inertial measurement signals on the same time grid as the TI signal.

The processing involving the TI measurement signal also includes generating, using the TI measurement signal, a pulmonary ventilation signal (see **FIG. 5,** for example) as is described above. The processing then further includes

determining $\varphi$ by iteratively varying $\varphi$ and determining a correlation between $\Delta a_x \cos(\varphi) + \Delta a_y \sin(\varphi)$ and the pulmonary ventilation signal until the correlation is maximized or otherwise attains a satisfactory value. The movement signal representative of the chest wall of the subject 104 is then configured as the linear combination of the first and second de-meaned inertial measurement signals that results from setting $\varphi$ to a value $\varphi_0$ that yield the maximal or otherwise satisfactory correlation.

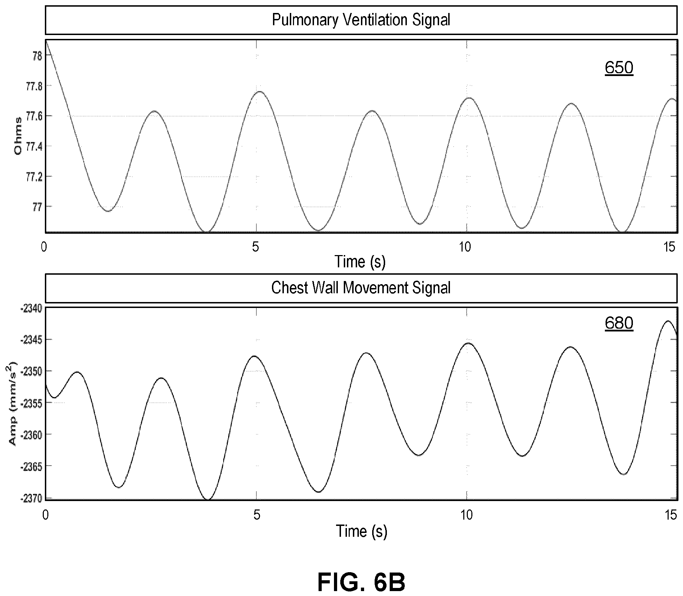

**FIG. 6B** presents a plot 650 of an example of the pulmonary ventilation signal, and a plot 680 of an example of a movement signal determined in accordance with aspects described herein. Both the pulmonary ventilation signal and the movement signal correspond to a same measurement period and are determined using multi-modal measurements performed by the wearable device 110 (**FIG. 1**) during the measurement period. The correlation between the illustrated pulmonary ventilation signal and movement signal is equal to 0.83.

Further, to determine the delay between mechanical onset of inspiration and onset of inspiration airflow, a server device of the multiple server devices 154 process the movement signal to determine times corresponding to respective mechanical onsets of inspiration in the breathing cycles contained in a measurement period. Those times can be referred to as marker points. The server device can determine the marker points by determining minima (troughs) and maxima (peaks) of the movement signal. For a particular minimum, the server device can determine a consecutive maximum, and also can determine a first value of the movement signal at the particular minimum. The server device can then identify a time at which a second value of the movement signal exceeds the first value by a defined amount. The defined amount is a defined fraction of a third value of the movement signals at the consecutive maximum. The server device can configure the identified time as a marker point. The server device can then identify another minimum and determines another marker point in the same fashion.

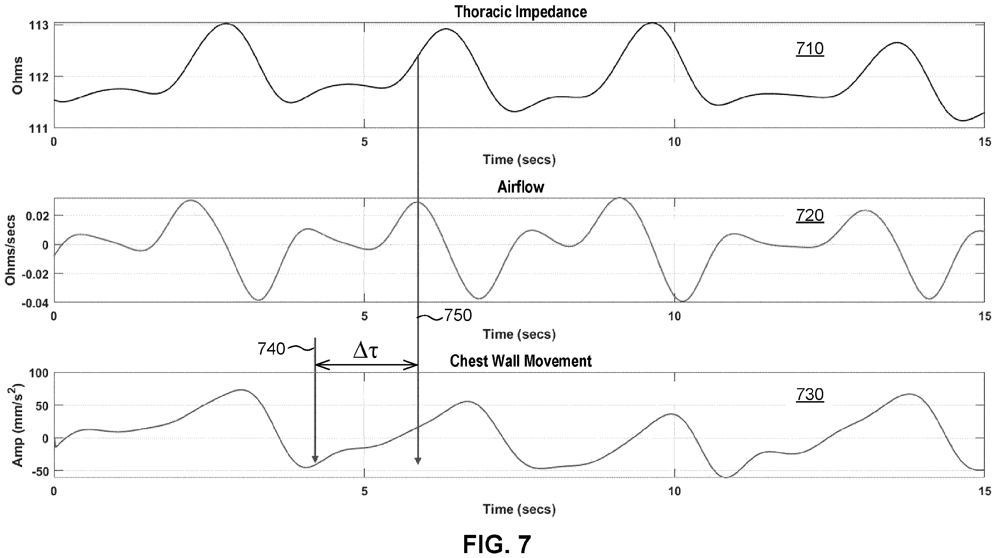

As part of determining the delay between mechanical onset of inspiration and onset of inspiration airflow, the server device also can generate an airflow signal based on the pulmonary ventilation signal that has been determined from a TI measurement signal for the measurement period. To that end, the server device can determine, over the measurement period, a first order derivative with respect to time of the pulmonary ventilation signal, resulting in the airflow signal. The server device can then determine times at which the airflow signal is maximal (or at peak) and can configure those times as marker points for peak positive airflow. Such marker points correspond to onset of inspiration airflow. Simply as an illustration, **FIG. 7** presents a plot 710 of de-meaned thoracic impedance signal corresponding to a subject (e.g., subject 104), and a plot 720 of airflow signal obtained from the de-meaned thoracic impedance signal in accordance with aspects described herein. **FIG. 7** also presents a plot 730 of chest wall movement signal corresponding to the subject.

It is noted that onset of inspiration flow may be obtained in other ways. For example, the server device may determine, using the respiration signals, the onset of inspiration airflow by determining, by applying a zero-crossing processing to the respiration signals, a time instant corresponding to air flow exceeding a threshold amount. In addition, the service device can then configure the time instant as the onset of inspiration airflow.

Further, the server device can identify a breathing cycle within the measurement period, and a first marker point and second marker point associated with the breathing cycle. The first marker point corresponds to mechanical onset of inspiration and the second marker point corresponds to onset of inspiration airflow. The server device can then determine the delay between mechanical onset of inspiration and onset of inspiration airflow as the difference between the second marker point and the first marker point. The server device can identify other breathing cycles within the measurement period, and can determine other such delays in the same fashion. Again with reference to **FIG. 7,** the server device can identify a first marker point 740 and a second marker point 750 corresponding, respectively, to mechanical onset of inspiration and onset of inspiration airflow. The delay between mechanical onset of inspiration and onset of inspiration airflow is the difference between the second marker point 750 and the first marker point 740, and is represented by $\Delta\tau$ in **FIG. 7.**

The processing platform 150 (**FIG. 1**) can use inertial measurement signals and TI measurement signals to evaluate an aggregate metric representative of respiratory effort. Such an aggregate metric is associated with respiration of a subject. Without intending to be bound by theory and/or modeling, respiratory effort can be defined as an amount of movement of the chest wall of a subject between the inhalation phase and the exhalation phase of a breath cycle. Further, because the extent of the movement of the chest wall can have a direct relation to the volume of air that enters and exits the lung, a more appropriate way to represent and measure respiratory effort is by normalizing the movement of the chest wall with respect to the volume of air that enters and exits the lungs (also referred to as volume of transacted air). Here, a direct relation among quantities refers to a relationship where a first change in one of the quantities directly causes a second change on another one of the quantities, where the second change is not necessarily linearly related with the first change.

As is described herein, the variation in pulmonary ventilation signal obtained from thoracic impedance measurements represent volume of transacted air. Accordingly, in some cases, an example of an aggregate metric that represents respiratory effort can be an amount of energy that is present in a movement signal relative to another amount of energy that is present in a pulmonary ventilation signal (also referred to as respiration signal). In some implementations, an amount of energy of a signal (e.g., movement signal or respiration signal) can be determined on a sample-by-sample basis. Thus, the processing platform 150 can determine an amount of energy $E[n]$ for a sample $n$ of the movement signal and another amount of energy $E'[n]$ for a sample $n$ of the respiration signal. The processing platform 150 can then evaluate a ratio of $E[n]$ to $E'[n]$ to determine a value of the aggregate metric. In other implementations, an amount of energy of the signal can be determined as an aggregate (an average or another function, for example) of energies of sample n and one or more immediately prior samples. Thus, the processing platform 150 can determine an amount of energy $E[n]$ for sample $n$ of the movement signals as an aggregate of energies of sample n and one or more immediately prior samples. The processing platform 150 also can determine another amount of energy $E'[n]$ for a sample of the respiration signal as an aggregate or energies of sample n and one or more immediately prior sample.

Further, or in other cases, movement of the chest wall of the subject can be normalized with respect to change in thoracic impedance. An expression for respiratory effort thus is:

$$R = \frac{\overline{a_h^2}}{\overline{\overline{Z}^2}} = \frac{\overline{a_x^2} + \overline{a_y^2}}{\overline{\overline{Z}^2}} \qquad\qquad \text{Eq. (1)}$$

where $\overline{x^2}$ denotes the variance of x, and $\overline{Z}$ is the de-meaned thoracic impedance signal, or the pulmonary ventilation signal, and $\overline{a_h}$ is the de-meaned in-plane acceleration described herein. As is described herein, the de-meaning of the thoracic impedance signal is performed in order to obtain a zero bias during correlation. Specifically, the respiratory pattern of a subject (e.g., subject 104) is embedded in the variation of the thoracic impedance signal and the DC value of such a signal has no information regarding respiratory dynamics. Because the three-axis accelerometer that can be included in the wearable device 110 generates three inertial measurement signals, another expression for the respiratory effort is:

$$R = \frac{\overline{a_h^2}}{\overline{\overline{Z}^2}} = \frac{\overline{a_x^2} + \overline{a_y^2} + \overline{a_z^2}}{\overline{\overline{Z}^2}} \qquad\qquad \text{Eq. (2)}$$

As such, another example of an aggregate metric that represents respiratory effort can be defined as a ratio of a variance of the movement signals and a variance of the respiration signals.

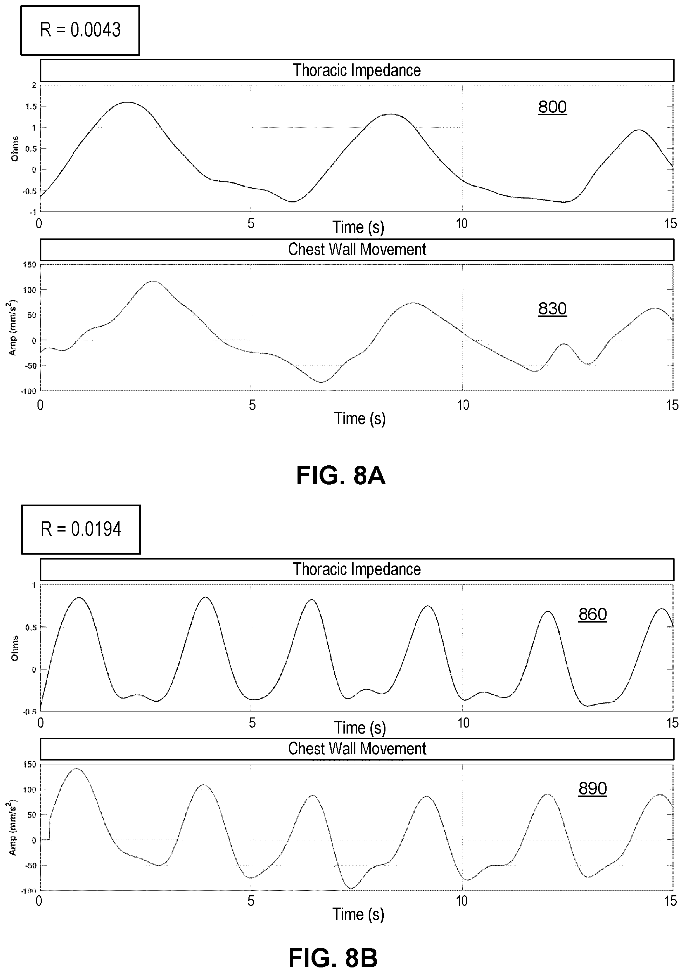

**FIG. 8A** presents a plot 800 of de-meaned thoracic impedance signal and a plot 830 of chest wall movement signal for a subject with stable COPD. The respiratory effort $R$ (Eq. (1)) that is calculated using such signals is equal to 0.0043. **FIG. 8B** presents a plot 860 of de-meaned thoracic impedance signal and a plot 890 of chest wall movement signal during an exacerbation of COPD in the same subject. The respiratory effort $R$ (Eq. (1)) that is calculated using such signals is equal to 0.0194.

The processing platform 150 (**FIG. 1**) can implement processes to determine a status change of a cardiopulmonary condition of the subject 104. The status change can be determined by tracking temporal changes in one or more metrics-direct metric(s), aggregate metric(s), or a combination thereof. The processes can include determining multiple values of a particular metric or a combination of metrics over a time interval. The time interval includes an analysis period that spans two or more test periods. Each test period spans multiple measurement periods, and includes at least one measurement period where the wearable device 110 has performed respective measurements and yields measurement signals. The analysis period may have a duration from at least one hour to a day or a week, with the duration being greater than another duration of a measurement period. In addition, or in some situations, the analysis period may span a few to many weeks to even a month or many months, the duration of the analysis period being greater than another duration of the measurement period.

Further, also as part of determining the status change, the processing platform 150, via one or more compute nodes, can monitor, using the multiple values, a time-dependence of the particular metric or the combination of metrics over the time interval. In some cases, monitoring such a time-dependence can include updating parameters defining a function (linear or otherwise) that fits currently determined values of the particular metric or each metric of the combination of metrics. In addition, or in other cases, monitoring such a time-dependence can include updating a parameter defining a rolling average of currently determined values of the particular metric or the combination of metrics. Regardless of how the time-dependence is monitored, the time-dependence of either the particular metric or a combination or metrics can convey a trend. The trend can be an upward trend, a downward trend, or an essentially constant trend. The upward trend can convey an exacerbation of the cardiopulmonary condition, whereas the downward trend can convey an improvement in the cardiopulmonary condition. The essentially constant trend indicates that the cardiopulmonary condition remains unchanged. Thus, by monitoring a single metric and/or combinations of metrics, the technologies described herein can provide detection and/or differentiation of cardiopulmonary exacerbations.

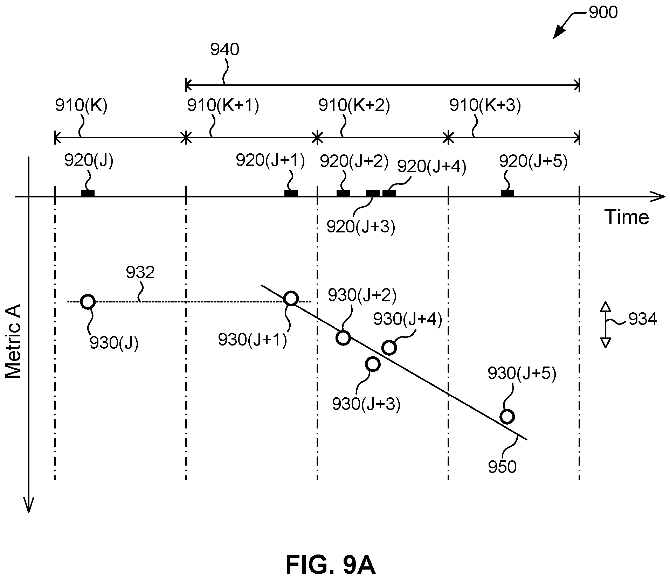

As an illustration, **FIG. 9A** presents a schematic plot 900 of a metric as a function of time over consecutive test periods, in accordance with aspects described herein. The metric may be any of the metrics in accordance with this disclosure and is denoted as "Metric A" in **FIG. 9A** simply for purposes of illustration. The ordinate represents the metric (e.g., $\rho_{EI}$) and the abscissa represents time. The consecutive test periods include a first test period 910(K), a second test period 910(K+1), a third test period 910(K+2), and a fourth test period 910(K+3). The first test period 910(K) and the second test period 910(K+1) include a single measurement period each. The third period 910(K+2) includes three measurement periods. The fourth test period 910(K+3) includes one measurement period. Each measurement period yields one or more measurement signals. The processing platform 150, via one or more server devices, can determine a value of the metric using the one or more measurement signals. Accordingly, the processing platform 150 determines a metric value 930(J) corresponding to the measurement period 920(J); a metric value 930(J+1) corresponding to the measurement period 920(J+1). The test period 910(K+2) includes a measurement period 920(J+2), a measurement period 920(J+3), and a measurement period 920(J+4). For such the test period 910(K+2), the processing platform 150 thus determines a metric value 930(J+2), a metric value 930(J+3), and a metric value 930(J+4). The test period 910(K+3) includes a measurement period 920(J+5), and the processing platform 150 determines a metric value 930(J+5). In the schematic plot 900, for each one of the metric values 930(J)-930(J+5), the processing platform 150, via one or more server devices, can determine a respective deviation value relative to a baseline value of the metric A. The baseline value can change over time. As is shown in **FIG. 9A,** each one of the metrics 930(J)-930(J+5) has a respective deviation value relative to the baseline value 932. Simply for purposes of illustration, a deviation value for the metric value 930(J+4) is indicated by an arrow 934.

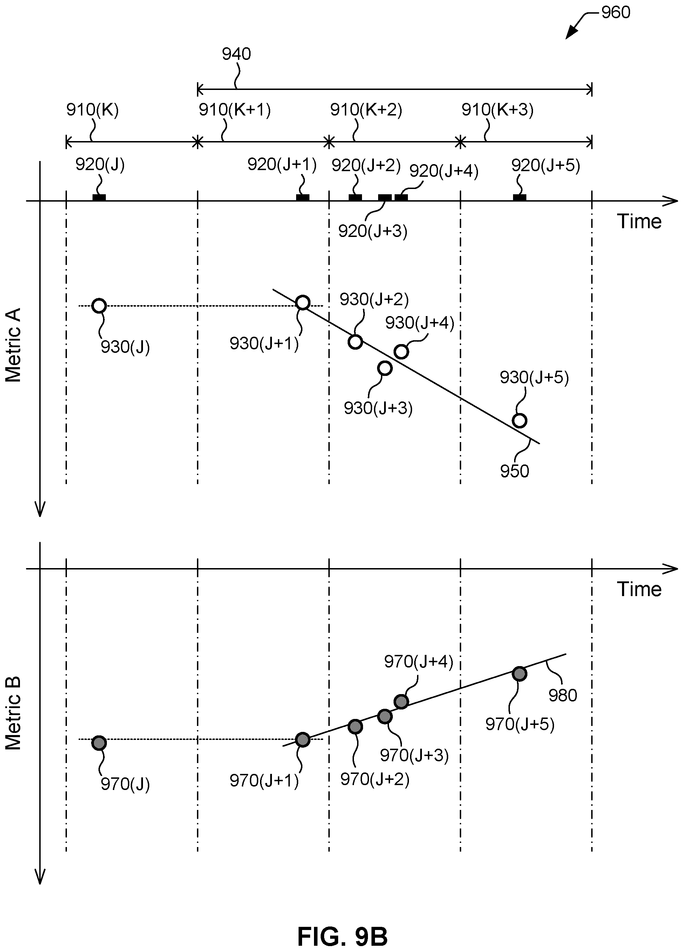

Continuing with the illustration, the processing platform 150 (**FIG. 1**), via one or more server devices, also can determine a time-dependence of the metric over an analysis period 940 (**FIG. 9**A, for example) that spans three test periods. To that end, in some cases, the processing platform 150 updates parameters defining a linear function that fits the metric values 930(J+1)-930(J+5) within the analysis period 940. As is shown in **FIG. 9A,** in one example, the updated parameters define a linear function 950 having a positive slope. The positive slope is indicative of an upward trend. Depending on the particular metric represented by the metric A, such an upward trend can convey an exacerbation of the cardiopulmonary condition. The magnitude of the positive slope can be indicative of the severity of the exacerbation. In addition, or in other cases, the processing platform 150 determines the time-dependence of the metric by updating a parameter indicative of a rolling average of the metric values 920(J+1)-920(J+5) over the analysis period 940. Regardless of how the time-dependence of the metric over the analysis period 940 is determined, the processing platform 150, via one or more server devices, can determine that the metric exhibits an upward trend.

In some cases, a combination of metrics and respective time dependences thereof can permit determining a status change in a cardiopulmonary condition (e.g., COPD) and differentiating the status change from another status change of another cardiopulmonary condition (e.g., CHF). As an example, both an acute exacerbation of COPD and an exacerbation of CHF can exhibit a drop in oxygen saturation level (SpO2). In AECOPD, the drop in SpO2 is more significant due to poor ventilation. Further, AECOPD may cause a small drop in thoracic impedance (due to excess mucus accumulation, for example) or may show an essentially unchanged thoracic impedance. In some cases, however, AECOPD may exhibit a significant increase in thoracic impedance. In contrast, in CHF, moderate levels of fluid accumulation affecting a small portion of alveoli do not impact SpO2 significantly as the body can increase respiration rate and, thus, hyperventilate other sections of unaffected alveoli, thereby maintaining oxygen saturation level during the exacerbation. In turn, thoracic impedance can exhibit a drop in magnitude even at moderate levels of exacerbation/decompensation. At significantly higher levels of exacerbation/decompensation where oxygen saturation levels cannot be maintained, the thoracic impedance can drop drastically. As another example, in a heart failure decompensation or a respiratory exacerbation, respiratory rate can increase with time and tidal volume can decrease as time increases.

**FIG. 9B** is schematic diagram 960 where a combination of a metric A and a metric B can be monitored as a function of time in order to determine a change in status of a cardiopulmonary condition. As is described herein, the processing platform 150, via one or more server devices, determines that the metric A increases with time over the analysis period 940. The processing platform 150 also determines that the metric B decreases as time increases over the analysis period 940. To that end, the processing platform 150 determines a metric value 970(J) corresponding to the measurement period 920(J); a metric value 970(J+1) corresponding to the measurement period 920(J+1); a metric value 970(J+2), a metric value 970(J+3), and a metric value 970(J+4) for the measurement periods 920(J+2)-920(J+4), respectively; and a metric value 970(J+5) corresponding to the measurement period 920(J+5). The processing platform 150, via one or more server devices, can then determine a time-dependence of the metric B over the analysis period 940. To that end, in some cases, the processing platform 150 updates parameters defining a linear function that fits the metric values 970(J+1)-970(J+5) within the analysis period 940. As is illustrated in **FIG. 9B,** the updated parameters define a linear function 980 having a negative slope. The negative slope is indicative of a downward trend. Depending on the metric B, such a downward trend individually can convey an exacerbation of the cardiopulmonary condition. Further, depending on the particular metrics A and B, it is the combination of the upward trend of metric A and the downward trend of metric B over the analysis period 940 that can be indicative of a change in the status of the cardiopulmonary condition. In one example, metric A is respiratory rate and metric B is tidal volume, and the combination of the trends represented by the linear function 950 and the linear function 980 is indicative an exacerbation CHF or COPD.

Subjects afflicted by both CHF and AECOPD present confounding symptoms to treating clinicians. Oftentimes, for these subjects, it is difficult to determine whether an exacerbation is due to a decompensation in CHF or increased severity of AECOPD. Symptoms such as breathlessness and cough are common in both conditions. Notwithstanding, in accordance with aspects of this disclosure, the origin of such an exacerbation can be elucidated by jointly monitoring, for example, SpO2 and thoracic impedance trends of such subjects. Subjects afflicted by CHF tend to maintain their SpO2 levels via hyper ventilation of lungs for mild to moderate levels of exacerbation due to fluid overload. At high level of fluid overload, however, SpO2 drops, and the afflicted subject exhibits a significant reduction in thoracic impedance. On the other hand, because AECOPD is an obstructive disorder of lungs and airways, AECOPD causes a more significant drop in SpO2 even under mild to moderate levels of exacerbation. In turn, thoracic impedance may remain relatively stable, may decrease by small amount due to excess mucous accumulation, or may show a significant increase due to air-trapping as that present in emphysema.

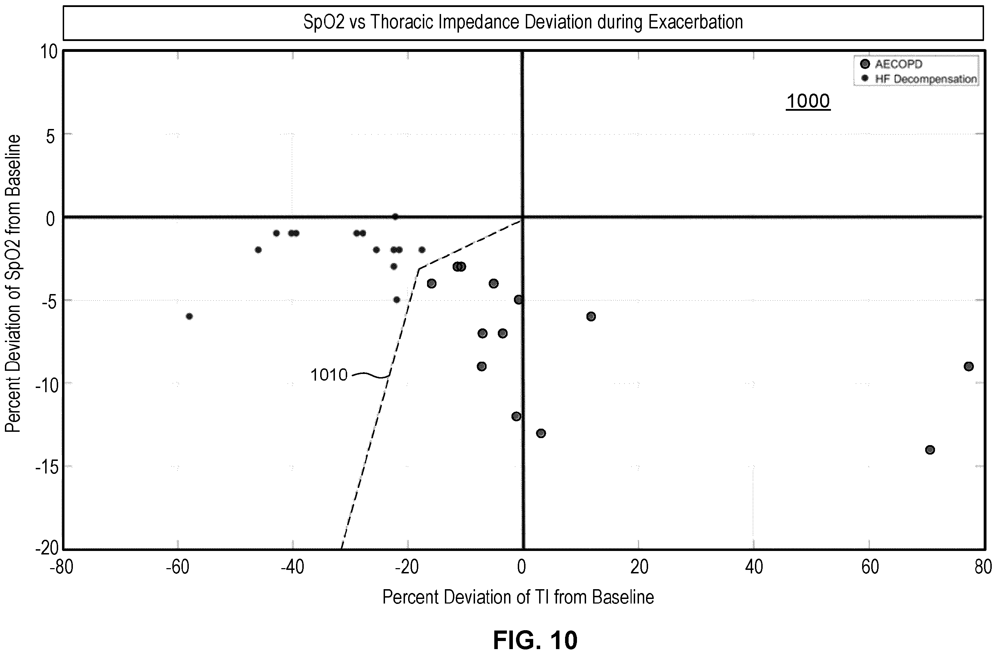

**FIG. 10** is a two-dimensional plot 1000 of SpO2 deviation from baseline versus thoracic impedance (TI) deviation from the baseline for patients having AECOPD and CHF exacerbation. The deviations are quantified in percent of baseline value. It can be readily visualized from the two-dimensional plot 1000 that the data occupy two different regions of the plot that can be well demarcated. Indeed, a classification line 1010 can be defined. Accordingly, the combination of respective values of SpO2 and thoracic impedance can classify or otherwise distinguish the type of exacerbation (either AECOPD or CHF).

Similar two-dimensional plots showing SpO2 deviation from baseline versus RR or minute ventilation (the product of RR and TV) deviation from baseline may also provide a differentiation diagram that permits distinguishing between AECOPD and CHF exacerbations. As mentioned, CHF patients with moderate decompensation may be able to maintain their SpO2 levels with increased ventilation. Yet, patients having AECOPD, specifically those with airway obstruction (or airway constriction), as is the case with chronic bronchitis, may be unable to maintain SpO2.

A three-dimensional plot with SpO2 deviation from baseline on a first axis, RR or minute ventilation deviation from baseline on a second axis, and thoracic impedance deviation from baseline on a third axis also can constitute a differentiation diagram that permits locating unique differentiated locations in three-dimensional space for clear differentiation of AECOPD or CHF decompensation. Such a differentiation diagram may include a two-dimensional classification boundary that distinguishing one type of exacerbation over the other type of exacerbation. Similarly, other combinations of metrics, such as $\rho_{EI}$, a breath morphology metric, and the like, with SpO2, thoracic impedance, and/or respiration rate can provide meaningful insights on the nature of an exacerbation.

Back to referring to **FIG. 1,** as is described herein, the processing platform 150 (via one or more of the server devices 154, for example) or a computing device can use values of a metric associated with respiration of a subject and/or respective values of a combination of such metrics in order to identify a status change of a cardiopulmonary condition of the subject 104. Such a metric can be a direct metric or an aggregate metric, in accordance with aspects described herein. In some cases, the processing platform 150 can identify, based on a change of a value relative to a past value of a metric, a status change of the cardiopulmonary condition. More specifically, the processing platform 150 can obtain multiple values of the metric for respective measurement periods within an analysis period. The processing platform 150 can monitor, over the analysis period, using the multiple values and another value of the metric, a time-dependence of the metric. That other value may have been determined at another measurement period. The processing platform 150 can then identify, based on the time-dependence, a status change of the cardiopulmonary condition.

In addition, or in other cases, the processing platform 150 or the computing device can identify, based on individual changes of respective values relative to respective past values of the metrics in a combination of metrics, a status change of the cardiopulmonary condition. More specifically, the processing platform 150 can obtain multiple respective values of the combination of metrics for respective measurement periods within an analysis period. The processing platform 150 can then monitor, over the analysis period, using the multiple respective values of the combination of metrics and other respective values of the combination of metrics, respective time-dependences of the metrics in the combination of metrics. Those other respective values may have been determined at another measurement period. The processing platform 150 can then identify, based on the respective time-dependences, a status change of the cardiopulmonary condition.

The example system 100 and other systems in accordance with the technologies described herein also include a computing device 170 that can be remotely located relative to both the processing platform 150 and the dwelling 106. A locale 172 of the computing device 170 can be a healthcare facility (e.g., a hospital) or a dwelling of an operator of the computing device 170, for example. The computing device 170 can be mobile, and thus, the locale 172 can change over time as the computing device 170 moves from one location to another.

The computing device 170 is depicted as a laptop computer simply for the sake of illustration. This disclosure is not limited in that respect. Indeed, the computing device 170 can be any device having computing resources that, individually or in combination, can execute processor-accessible instructions and can cause a display device to display information. The processor-accessible instructions may be processor-executable program code. In some cases, the processor-executable program code may be embodied in software libraries, application programming interfaces (APIs), processor-executable modules, and the like. The display device may be integrated into the computing device 170 or functionally coupled therewith. Further, the computing device 170 may be mobile (handheld or otherwise) or pseudo-stationary. Besides a laptop computer, examples of the computing device 170 include a smartphone, a phablet device, a tablet computer, or similar apparatuses.

The computing device 170 is functionally coupled or otherwise configured to communicate with the processing platform 150 via at least one of the network(s) 140. The computing device 170 can be associated with, or otherwise operated by, a clinician. Thus, in some cases, the computing device 170 may be referred to as a clinician device. In other cases, the computing device 170 can be associated with, or otherwise operated by, a caregiver or the subject 104. Thus, in some cases, the computing device 170 may be referred to as a caregiver device. In yet other cases, the computing device 170 can be a computing device within a hospital computing system. Further, or in some scenarios, another computing device (not shown in **FIG. 1**) also can be functionally coupled or otherwise configured to communicate with the processing platform 150. That other computing device can be associated with, or otherwise operated by, an agent of a health insurance provider. The agent can be a human or an autonomous bot executed by such a computing device. The clinician can be involved in providing care for cardiopulmonary condition of the subject 104. The agent can be involved in making decisions related to financial coverage of such care. Accordingly, the computing device 170 and the other computing device (not shown in **FIG. 1**) can receive information related to a status of the cardiopulmonary condition of the subject 104 and/or a change in the status of the cardiopulmonary condition of the subject 104. Because the change in status of a cardiopulmonary condition may warrant medical intervention, such as transportation to a hospital and/or hospitalization, the contemporaneous availability of the information related to one or both of the change in the status of the cardiopulmonary condition or the change itself can avoid a potentially fatal delay in care. Thus, the systems, devices, and techniques, individually or combination, in accordance with embodiments of the technologies described herein provide a clear improvement over commonplace technologies for the management of cardiopulmonary conditions.

At least a portion of the information supplied by the processing platform 150 to the computing device 170 can be formatted as an electronic message that can be presented by a software application, or another type of program code, configured to execute in the computing device 170. The computing device 170, via the software application, can receive and present the information. The information can be presented in at least one of multiple user interfaces 174 that the computing device 170 can present in response to the software application being executed. In some cases, the information can be presented in a user interface that is presented in a display device integrated into the computing device 170 or functionally coupled thereto. In other cases, the information can be presented as an overlay (e.g., a push-up message) in a home interface presented in the display device, in response to the software application being executed. In one example, the processing platform 150 can cause the computing device 170 to present one or more markings indicative of a status change of the cardiopulmonary condition-e.g., a transition from historical baseline to an exacerbated state of the cardiopulmonary condition of the subject 104. The marking(s) can be presented in the user interface or the overlay, and can be formatted to convey the nature of the status change and, in some cases, identify the subject 104. For example, for a transition from historical baseline to a distress state, the marking(s) can be formatted to conspicuously convey such a transition.

Simply as an illustration, **FIG. 2A** presents an example user interface 202 that can be part of the multiple user interfaces 174 presented by the computing device 170. Because the computing device 170 can have access to information corresponding to multiple wearable devices and subjects besides the wearable device 110 and the subject 104, the example user interface 202 includes a first selectable visual element 204 that, in response to being selected, causes the computing device 170 (i) to obtain data identifying wearable devices that are available for monitoring a respective subject and (ii) to present a listing of the wearable devices (including the wearable device 110). Each item in the listing also can be selectable and, in response to being selected, causes the example user interface 202 to include a visual element 206 identifying a selected wearable device (e.g., wearable device 110). The example user interface 202 also includes a second selectable visual element 208 that, in response to being selected, cause the computing device 170 to establish a connection (e.g., a communication session) with the selected wearable device.

The example user interface 202 also includes a visual element 210 identifying the subject 104 corresponding to the wearable device 110. For example, the visual element 210 can include text and/or other markings identifying the subject. The example user interface 202 also includes a second visual element 220 indicating a value of SpO2, a plot 230 of ECG data over a measurement period 235, and a plot 240 of thoracic impedance over a measurement period (e.g., 30 s, 60 s, 120 s). In some cases, the example user interface 202 also includes a first selectable visual element 250 and a third selectable visual element 260. Selection of the first selectable visual element 250 causes the computing device 170 to direct the wearable device 110 to generate measurement signals. Selection of the fourth selectable visual element 260 causes the computing device 170 to direct the wearable device to stop the generation of measurement signals. The disclosure is, of course, not limited to the visual elements and the layout of the visual elements shown in the example user interface 202. Other types of visual elements can be presented in a UI of the multiple user interfaces 174 and/or other layouts of visual elements can be implemented.

With further reference to **FIG. 1,** the computing device 170 also may be functionally coupled or otherwise configured to communicate with the user device 160 via at least one of the network(s) 140. In some configurations, the user device 160 may be part of the example system 100 or other systems in accordance with aspects of the technologies described herein. The user device 160 may be owned, leased, and/or operated by the subject 104. An aid assisting the subject with performing measurements also may operate the user device 160. The aid can be, for example, a family member or another type of caregiver, such as a nurse, a nurse assistant, nursing home staff, or similar support personnel. The user device 160 can be configured with a software application, or another type of program code, to exchange information with the computing device 170 and the wearable device 110. As such, the computing device 170, via the software application configured in the computing device 170, can provide an indication of an action to be executed by the subject 104 in response to a particular value of a metric being monitored and/or a status change in the cardiopulmonary condition of the subject 104. The indication can be, for example, an instruction or another type of directive according to a particular control protocol. The user device 160, via the software application configured in the user device 160, can receive such an indication and, in response, can present a message directing the subject 104 to execute the action. The message can be presented in at least one of multiple user interfaces 164 that the user device 160 can present in response to the software application being executed. The message can be a push-up message or a recorded voice message, for example. The push-up message can be part of or can form one of the multiple user interfaces 164. In some cases, the push-up message may be presented on a home screen or lock screen of the user device 160. The recorded voice message can be selected from a menu of voice messages retained within the user device 160 or can be generated by the user device 160, via the software application. The voice messages may be retained within a memory device integrated into the user device 160. Thus, the computing device 170 can prompt, based on the particular value of the metric or particular values of respective metrics in a combination of metrics, the subject 104, a clinician, and/or a caregiver to cause the wearable device 110 to generate additional measurement signals.

In addition, or in some cases, to provide an indication of an action to be executed by the subject 104, the computing device 170 can cause transmission of an electronic communication to a clinician device and/or a second electronic communication to a caregiver device. The electronic communication and the second electronic communication both include the indication. Each of those electronic communication may be one of an email, a text message, a telephonic call, or a videoconference call. As is described herein , the indication may be provided in response to a particular value of a metric being monitored, a particular value of a combination of metrics, particular respective values of metrics in the combination of metrics, and/or a status change in the cardiopulmonary condition of the subject 104.

Simply as an illustration, a variant of the example user interface 202 (**FIG. 2A**) can be part of the multiple user interfaces 164 presented by the user device 160. For example, in cases where the user device 160 is only functionally coupled or otherwise configured to communicate with the wearable device 110, the variant of the example user interface 202 can exclude the first selectable visual element 204, the visual element 206, and the second selectable visual element 208. The disclosure is, of course, not limited to the visual elements and the layout of the visual elements shown in the example user interface 202 or variants thereof. Indeed, the user device 160 can present other types of visual elements in a UI of the multiple user interfaces 164 and/or other layouts of visual elements can be implemented.

In some cases, the type of action directed by the computing device 170 is associated with the nature of the status change. As an example, for a transition from historical baseline of a metric, or combination of metrics, to a distress state, the action can include proceeding to an emergency room, contacting a primary physician, and or contacting an aid. As another example, based on values of a metric or a combination of metrics, the action can include causing the wearable device 110 to generate additional measurement signals. In some implementations, the computing device 170 can cause the wearable device 110 to generate additional measurement signals as part of an additional measurement period, without intervention of the subject 104. In those implementations, the user device 160 can relay a directive to generate additional measurement signals or, in some cases, the user device 160 can generate such a directive and can then send the directive to the wearable device 110. As an illustration, with reference to **FIG. 9A,** the measurement periods 920(J+3) and 920(J+4) can be caused by the computing device 170 in response to the metric A value 930(J+2) being greater than metric A value 930(J+1). Other actions are of course contemplated.

With further reference to **FIG. 1,** as is described herein, the user device 160 can be functionally coupled or otherwise configured to communicate with the wearable device 110. The user device 160 also can be functionally coupled or otherwise configured to communicate with the processing platform 150 via at least one of the network(s) 140. Accordingly, in some configurations, the user device 160 can provide at least some of the functionality of the computing device 170, described herein in accordance with aspects of this disclosure.

The software application, or another type of program code, that may be configured in the user device 160 can control the manner of performing measurements using the wearable device 110. To that end, as is described herein, in response to the software application being executed, the user device 160 can present a user interface that permits configuring a measurement session, e.g., number of measurements to be performed (or signal samples to be obtained) during a measurement period. A measurement session may be referred to as a measurement cycle. The user interface may be one of the multiple user interfaces 164.

The configuration of a measurement session may be guided by the user device 160, via the software application. For example, the user device 160, via the software application, can prompt the subject 104 or an aid to execute an action to configure an attribute of the measurement session. An example of an attribute of the measurement session is placement of the wearable device 110. As such, the user device 160, via the software application, can prompt the subject 104 or aid to adjust placement of the wearable device 110 by causing the wearable device 110 to vibrate, continuously or intermittently, until proper placement is achieved. Specifically, as placement of the wearable device 110 is adjusted, the user device 160 can determine if placement of the wearable device 110 on the torso of the subject 104 satisfies a placement rule. To determine placement, for example, the user device 160 can receive measurement signals from an inertial sensor that is part of the wearable device 110. For some placements, the user device 160 can determine that placement of the wearable device 110 on the torso of the subject 104 fails to satisfy the placement rule. In response to such a determination, the user device 160 can direct a haptic device integrated into the wearable device 110 to cause vibration of the wearable device 110. In some cases, the haptic device is included in the I/O interfaces 130. In other cases, the haptic device may be a standalone component coupled with the processor(s) 122, the memory device(s) 126, the I/O interfaces 130, and/or the network adapter(s) 134. The user device 160 can continue receiving measurement signals from the inertial sensor, and can thus continue monitoring placement of the wearable device 110. As part of such monitoring, at a time after an initial improper placement of the wearable device 110, the user device 160 can determine that an adjusted placement of the wearable device 110 on the torso of the subject 104 satisfies the placement rule. In response, the user device 160 can direct the haptic device to cease vibration of the wearable device 110. Additionally, or as an alternative, the user device 160 can present a notification (e.g., visual and/or aural) indicating that the wearable device 110 is properly placed on the torso of the subject 104. In addition, or in some configurations, the wearable device 110 itself can evaluate proper placement of the wearable device 110, in accordance with aspects described herein. To that end, the wearable device 110 may implement the operations described hereinbefore that the user device 160 can implement for placement of the wearable device 110.

Further, or in some cases, the software application or other program code that is configured in the user device 160 can control end-user activities that prepare the subject 104 for a measurement using the wearable device 110. To that end, in some cases, in response to the software application being executed, the user device 160 can present a user interface including an indication of an action to be executed by the subject prior to a measurement (or the measurement period associated with the measurement). The user interface may be one of the user interfaces 164. The indication can include, for example, one or more visual elements identifying the action to be executed, and in some cases, providing a tutorial on how to perform the action. The visual element(s) can include text or other markings, a still image, a video segment, or a combination thereof. The action includes, for example, performing one or more movements to stimulate or otherwise stress a cardiopulmonary system of the subject 104. A movement to be performed can be customized to the level of physical fitness and/or mobility of the subject 104. Information on the level of physical fitness and/or mobility of the subject 104 can be retained in a memory device that is integrated into the user device 160. A user profile can retain the information on the level of physical fitness and/or mobility of the subject 104. Other data structures besides a user profile may be used. In addition, or in some cases, the information may be retained in on the level of physical fitness and/or mobility of the subject 104 can be retained in a storage node (e.g., one of the server devices 154) of the processing platform 150. Examples of movements to be performed include walking in place for a defined period, jogging in place for a defined period, climbing a number of stairs steps, performing a compound movement (such as lunges, squats, or sit-to-stand maneuvers); rocking torso and arms; and the like.

Furthermore, or in some cases, the user device 160 (via the software application, for example) also can present one or more other user interfaces that display observed data from measurements. The observed data can include, for example, values of thoracic impedance at one or more time, values of SpO2, and the like. As is described herein, **FIG. 2A** illustrates an example of a user interface that presents observed data for a subject.

With further reference to **FIG. 1,** the user device 160 (via the software application, for example) can determine that a reporting rule has been satisfied and, in response, can cause presentation of a patient reported outcome (PRO) questionnaire associated with a cardiopulmonary condition of the subject 104. The PRO questionnaire can be presented as a single user interface or a series of user interfaces. The single user interface and the series of user interfaces can be part of the multiple user interfaces 164. To cause presentation of PRO questionnaire, the user device 160 can cause a display device to present the PRO questionnaire. The display device can be integrated into the user device 160 or can be functionally coupled thereto. The computing device 170 also can provide such a functionality.

To determine that a reporting rule has been satisfied, the user device 160 can evaluate, according to the reporting rule, data associated with monitoring the status of a cardiopulmonary condition of the subject 104. In one example, the reporting rule dictates that a PRO questionnaire is to be presented upon or after a defined number of measurement sessions have been completed. Thus, the data that may evaluate include data defining a number of measurement sessions currently completed. In addition, or in another example, the reporting rule dictates that a PRO questionnaire is to be presented after a defined number of successive measurements have yielded values of a metric indicative of the status of the cardiopulmonary condition that are greater than a baseline value. Further, or in yet another example, the reporting rule dictates that a PRO is to be presented in response to input information received by the user device 160 indicating that a subject is ready to response to the PRO questionnaire. In some instances, a result of such an evaluation indicates that the reporting rule is satisfied. In those instances, the user device 160 causes presentation of the PRO questionnaire. The computing device 170 also can provide such a functionality.

The user device 160 can receive answers to the PRO questionnaire and can send one or more of the answers to the computing device 170 and/or the processing platform 150.

As is described herein, the user device 160 and the computing device 170 may be configured with a software application that provides functionality described herein in connection with monitoring and identification of a status change of a cardiopulmonary condition of a subject. A group of the server devices 154 within the processing platform 150 can host the software application. Thus, the user device 160, the computing device 170, and any other computing device configured with the software application can implement the functionality of the server device 154 via the software application (e.g., via API and/or other types of libraries and related function calls.

As is described herein, it is noted that this disclosure is not limited to the structure of the sensor devices 118 shown in **FIG. 1.** The sensor devices 118 may include fewer or more types of sensor devices than those shown in **FIG. 1,** and the functionality described herein in connection with monitoring and identification of status change of a cardiopulmonary condition still can be achieved.

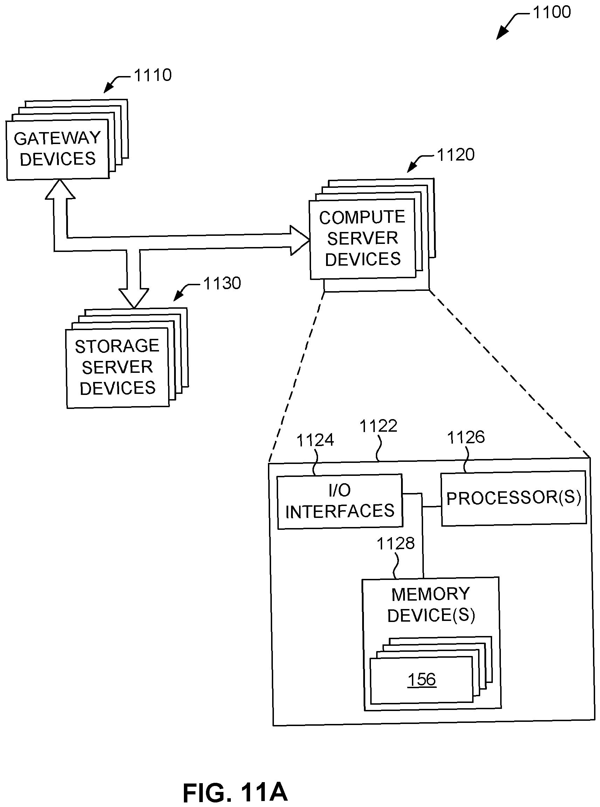

**FIG. 11A** is a schematic block diagram of an example of a computing system 1100 that can implement analysis of multi-modal measurement signals generated by the wearable device 110 (**FIG. 1**), in accordance with one or more aspects of this disclosure. The computing system 1100 can embody, or can be part of, the processing platform 150 described herein in connection with example systems and techniques of this disclosure. Accordingly, the example computing system 1100 can provide at least some of the functionality described herein in connection with monitoring status changes of a cardiopulmonary condition using multi-modal measurement signals in accordance with aspects described herein.

As mentioned, the processing platform 150 includes multiple server devices 154. The multiple server devices 154 can be arranged in a cloud architecture. As such, the example computing system 1100 includes two types of server devices: Compute server devices 1120 and storage server devices 1130. A subset of the compute server devices 1120, individually or collectively, can host various modules that permit implementing the analysis included in monitoring status changes of a cardiopulmonary condition using multi-modal measurement signals in accordance with aspects described herein. Thus, the subset of compute server devices 1120 can operate in accordance with functionality described herein in connection with monitoring status changes of a cardiopulmonary condition using multi-modal measurement signals. The architecture of each of the compute server devices 1120, including the compute server device 1122, includes multiple input/output (I/O) interfaces 1124, one or more processors 1126, one or more memory devices 1128, and a bus architecture that functionally couples the processor(s) and the memory device(s). In some cases, the compute server device 1122 can store such modules in at least one of such memory device(s) 1128. At least the subset of the compute server devices 1120 can be functionally coupled or otherwise configured to communicate with one or multiple ones of the storage server devices 1130. The coupling can be direct or can be mediated by at least one of the gateway devices 1110. The storage server devices 1130 include data and/or metadata that can be used to implement the functionality described herein in connection with monitoring status changes of a cardiopulmonary condition using multi-modal measurement signals.

Each one of the gateway devices 1110 can include one or multiple processors functionally coupled or otherwise configured to communicate with one or multiple memory devices that can retain application programming interfaces (APIs) and/or other types of program code for access to the compute server devices 1120 and storage server devices 1130. Such access can be programmatic, via a defined function call, for example. The subset of the compute server devices 1120 that host one or a combination of modules that can use API(s) supplied by the gateway devices 1110 in order to provide results of implementing the functionalities described herein in connection with monitoring status changes of a cardiopulmonary condition using multi-modal measurement signals in accordance with aspects described herein.

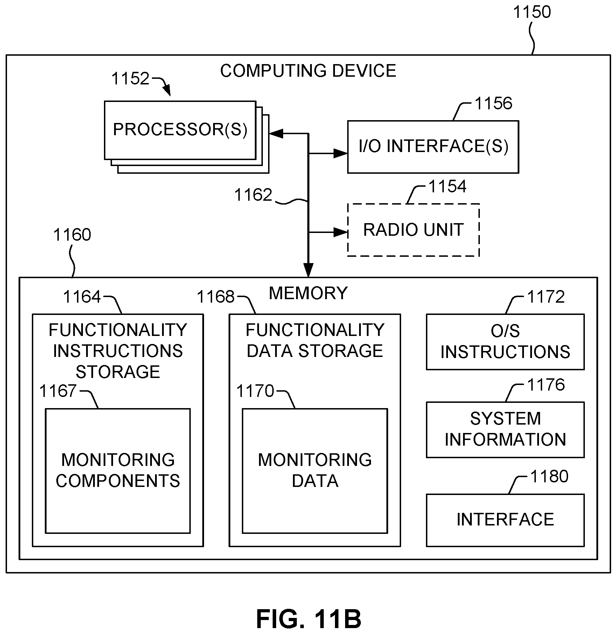

**FIG. 11B** illustrates an example of a computing device that can provide various functionalities of monitoring and identification of changes in status of a cardiopulmonary condition of a subject in accordance with aspects of this disclosure. In some cases, the example computing device 1150 can embody the user device 160 (**FIG. 1**). In other cases, the example computing device 1150 can embody the computing device 170. In yet other cases, the example computing device 1150 can embody another type of computing device in accordance with aspects of this disclosure, such as a caregiver device. The example computing device 1150 can provide such functionalities in response to execution of one or more software components retained within the computing device 1150. Such component(s) can render the example computing device 710 a particular machine for monitoring and identification of changes in status of a cardiopulmonary condition of a subject, among other functional purposes that the example computing device 1150 may have. A software component can be embodied in or can comprise one or more processor-accessible instructions, e.g., processor-readable and/or processor-executable instructions. In one scenario, at least a portion of the processor-accessible instructions can embody and/or can be executed to perform at least a part of one or more of the techniques described herein. The one or more processor-accessible instructions that embody a software component can be arranged into one or more program modules, for example, that can be compiled, linked, and/or executed at the example computing device 1150 or other computing devices. Generally, such program modules comprise computer code, routines, programs, objects, components, information structures (e.g., data structures and/or metadata structures), etc., that can perform particular tasks (e.g., one or more operations) in response to execution by one or more processors 1152 integrated into the example computing device 1150. The software component(s) may form a software application for monitoring and identification of changes in status of a cardiopulmonary condition of a subject, in accordance with this disclosure, as is described herein in connection with the user device 160 and other computing devices.

The various example aspects of the disclosure can be operational with numerous other general purpose or special purpose computing system environments or configurations. Examples of well-known computing systems, environments, and/or configurations that can be suitable for implementation of various aspects of the disclosure in connection contactless monitoring of vital signs can include personal computers; server computers; laptop devices; handheld computing devices, such as mobile tablets or laptop computers; wearable computing devices; and multiprocessor systems. Additional examples can include network personal computers (PCs), mainframe computers, blade computers, programmable logic controllers, embedded systems, distributed computing environments that comprise any of the above systems or devices, and the like.

As is illustrated in **FIG. 11B**, the example computing device 1150 includes one or multiple processors 1152, one or multiple input/output (I/O) interfaces 1156, one or more memory devices 1160 (referred to as memory 1160), and a bus architecture 1162 (referred to as bus 1162) that functionally couples various functional elements of the example computing device 1150. The example computing device 1150 can include, optionally, a radio unit 1154. The radio unit 1154 can include one or more antennas and a communication processing unit that can permit wireless communication between the example computing device 1150 and another device, such as a remote computing device, a display device, and/or a remote sensor device. The bus 1162 can include at least one of a system bus, a memory bus, an address bus, or a message bus, and can permit the exchange of information (data and/or signaling) between the processor(s) 1152, the I/O interface(s) 1156, and/or the memory 1160, or respective functional elements therein. In some cases, the bus 1162 in conjunction with one or more internal programming interfaces 1180 (also referred to as interface 1180) can permit such exchange of information. In cases where the processor(s) 1152 include multiple processors, the example computing device 1150 can utilize parallel computing.

The I/O interface(s) 1156 can permit communication of information between the example computing device 1150 and an external device, such as another computing device, a display device, or similar device. Such communication can include direct communication or indirect communication, such as the exchange of information between the example computing device 1150 and the external device via a network or elements thereof. As illustrated, the I/O interface(s) 1156 can include one or more of network adapter(s), peripheral adapter(s), and display unit(s). Such adapter(s) can permit or otherwise facilitate connectivity between the external device and one or more of the processor(s) 1152 or the memory 1160. For example, the peripheral adapter(s) can include a group of ports, which can include at least one of parallel ports, serial ports, Ethernet ports, V.35 ports, or X.21 ports. In certain aspects, the parallel ports can comprise General Purpose Interface Bus (GPIB), IEEE-1284, while the serial ports can include Recommended Standard (RS)-232, V.11, Universal Serial Bus (USB), FireWire or IEEE-1394.

The I/O interface(s) 1156 can include a network adapter that can functionally couple the example computing device 1150 to one or more remote computing devices, display devices, or sensors devices (not depicted in **FIG. 11B**) via one or more traffic and signaling pipes that can permit or otherwise facilitate the exchange of traffic and/or signaling between the example computing device 1150 and such one or more remote computing devices or sensors. Such network coupling provided at least in part by the network adapter can be implemented in a wired environment, a wireless environment, or both. The information that is communicated by the network adapter can result from the implementation of one or more operations of a method or a combination of methods in accordance with aspects of this disclosure. The I/O interface(s) 1156 can include more than one network adapter in some cases.

In addition, or in some cases, depending on the architectural complexity and/or form factor the example computing device 1150, the I/O interface(s) 1156 can include a user-device interface unit that can permit control of the operation of the example computing device 1150, or can permit conveying or revealing the operational conditions of the computing device 710. The user-device interface can be embodied in, or can include, a display unit. The display unit can include a display device that, in some cases, has touch-screen functionality. In addition, or in some cases, the display unit can include lights, such as light-emitting diodes, that can convey an operational state of the computing device 1150.

The bus 1162 can have at least one of several types of bus structures, depending on the architectural complexity and/or form factor the computing device 1150. The bus structures can include a memory bus or a memory controller, a peripheral bus, an accelerated graphics port, and a processor or local bus using any of a variety of bus architectures. As an illustration, such architectures can comprise an Industry Standard Architecture (ISA) bus, a Micro Channel Architecture (MCA) bus, an Enhanced ISA (EISA) bus, a Video Electronics Standards Association (VESA) local bus, an Accelerated Graphics Port (AGP) bus, a Peripheral Component Interconnect (PCI) bus, a PCI-Express bus, a Personal Computer Memory Card International Association (PCMCIA) bus, a Universal Serial Bus (USB), and the like.

The example computing device 1150 can include a variety of computer-readable media. Computer-readable media can be any available media (transitory and non-transitory) that can be accessed by a computing device. In one aspect, computer-readable media can comprise computer non-transitory storage media (or computer-readable non-transitory storage media) and communications media. Examples of computer-readable non-transitory storage media include any available media that can be accessed by the example computing device 1150, including both volatile media and non-volatile media, and removable and/or non-removable media. The memory 1160 can include computer-readable media in the form of volatile memory, such as random access memory (RAM), and/or non-volatile memory, such as read-only memory (ROM).

The memory 1160 can include functionality instructions storage 1164 and functionality data storage 1168. The functionality instructions storage 1164 can include processor-accessible instructions that, in response to execution (by at least one of the processor(s) 1152, individually or in combination, for example), can implement one or more of the functionalities of this disclosure in connection with monitoring and identification of changes in status of a cardiopulmonary condition of a subject. The processor-accessible instructions can embody, or can include, one or more software components illustrated as monitoring components 1167. Execution of at least one component of the monitoring components 1167 can implement one or more of the methods, individually or in combination, in accordance with aspects described herein. Such execution can cause a processor (e.g., one of the processor(s) 1152) that executes the at least one component to carry out at least a portion of the methods disclosed herein.

A processor of the processor(s) 1152 that executes at least one of the monitoring components 1167 can retrieve data from or retain data in one or more memory elements 1170 in the functionality data storage 1168 in order to operate in accordance with the functionality programmed or otherwise configured by the monitoring components 1167. The one or more memory elements 1170 may be referred to as monitoring data 1170. Such information can include at least one of code instructions, data structures, or similar. For example, at least a portion of such data structures can define one or more of the various time series of values for signals in accordance with aspects of this disclosure.

The interface 1180 (e.g., an application programming interface) can permit or facilitate communication of data between two or more components within the functionality instructions storage 1164. The data that can be communicated by the interface 1180 can result from implementation of one or more operations in a method or a combination of methods of this disclosure. In some cases, one or more of the functionality instructions storage 1164 or the functionality data storage 1168 can be embodied in or can comprise removable/non-removable, and/or volatile/non-volatile computer storage media.

At least a portion of at least one of the monitoring components 1167 or the monitoring data 1170 can program or otherwise configure one or more of the processor(s) 1152 to operate at least in accordance with the functionality described herein. One or more of the processor(s) 1152, individually or in combination, can execute at least one of the monitoring components 1167, and also can use at least a portion of the data in the functionality data storage 1168 in order to provide monitoring and identification of changes in status of a cardiopulmonary condition of a subject in accordance with one or more aspects described herein. In some cases, the functionality instructions storage 1168 can embody or can include a computer-readable non-transitory storage medium having computer-accessible instructions that, in response to execution, cause at least one processor (e.g., one or more of the processor(s) 1152) to perform a group of operations comprising the operations or blocks described in connection with example methods disclosed herein.

In addition, the memory 1170 can include processor-accessible instructions and information (e.g., data, metadata, and/or program code) that permit or facilitate the operation and/or administration (e.g., upgrades, software installation, any other configuration, or the like) of the example computing device 1150. Accordingly, as illustrated, the memory 1160 can include a memory element 1172 (labeled operating system (O/S) instructions 1172) that contains one or more program modules that embody or include one or more operating systems, such as Windows operating system, Unix, Linux, Symbian, Android, Chromium, and substantially any OS suitable for mobile computing devices or tethered computing devices. In one aspect, the operational and/or architectural complexity of the example computing device 1150 can dictate a suitable O/S. The memory 1160 also includes system information storage 1176 having data, metadata, and/or program code that permits or facilitates the operation and/or administration of the example computing device 1150. Elements of the O/S instructions 1172 and the system information storage 1176 can be accessible or can be operated on by at least one of the processor(s) 1152.

It should be recognized that while the components retained in the functionality instructions storage 1164 and other executable program components, such as the O/S instructions 1172, are illustrated herein as discrete blocks, such software components can reside at various times in different memory components of the example computing device 1150, and can be executed by at least one of the processor(s) 1152, individually or in combination.

The example computing device 1150 can include a power supply (not shown), which can power up components or functional elements within such devices. The power supply can be a rechargeable power supply, e.g., a rechargeable battery, and it can include one or more transformers to achieve a power level suitable for the operation of the example computing device 1150 and components, functional elements, and related circuitry therein. In some cases, the power supply can be attached to a conventional power grid to recharge and ensure that such devices can be operational. To that end, the power supply can include an I/O interface (e.g., one of the interface(s) 1176) to connect to the conventional power grid. In addition, or in other cases, the power supply can include an energy conversion component, such as a solar panel, to provide additional or alternative power resources or autonomy for the example computing device 1150.

In some scenarios, the example computing device 1150 can operate in a networked environment by utilizing connections to one or more remote computing devices and/or sensor devices (not depicted in **FIG. 11B**). As an illustration, a remote computing device can be a personal computer, a portable computer, a server, a router, a network computer, a peer device or other common network node, and so on. As described herein, connections (physical and/or logical) between the example computing device 1150 and a remote computing device or sensor can be made via one or more traffic and signaling pipes, which can comprise wired link(s) and/or wireless link(s) and several network elements (such as routers or switches, concentrators, servers, and the like) that form a local area network (LAN), a wide area network (WAN), and/or other networks (wireless or wired) having different footprints.

Other architectures of systems to monitor status changes of a cardiopulmonary condition also can be implemented. As an illustration, **FIG. 12** is a schematic block diagram of an example of a system 1200 to monitor status changes of a cardiopulmonary condition also can be implemented, in accordance with one or more aspects of this disclosure. In the example system 1200, the structure of the wearable device 110 can be simplified relative to the example system 100 (FIG. 1). To that end, a base station device 1210 functionally coupled or otherwise configured to communicate with the wearable device 110 can provide control functionality for the wearable device 110. The base station device 1210 also can be functionally coupled or otherwise configured to communicate with the processing platform 150, and can provide data and signaling to the processing platform 150. The data can be indicative of measurement signals generated by the multiple sensor devices 118. The base station device 1210 also can be functionally coupled or otherwise configured to communicate with the user device 160 and the computing device 170 via the network(s) 140.

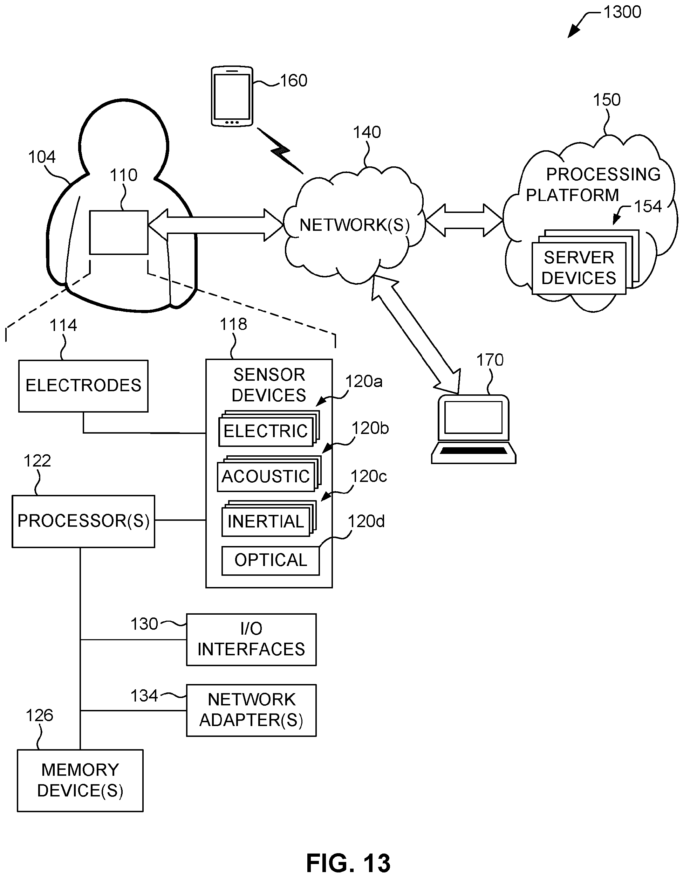

As another illustration, **FIG. 13** is a schematic block diagram of an example of a system 1300 to monitor status changes of a cardiopulmonary condition also can be implemented, in accordance with one or more aspects of this disclosure. In the example system 1300, the wearable device 110 can be functionally coupled or otherwise configured to communicate with the processing platform 150 via the network(s) 140. And can provide data and signaling to the processing platform 150. The data can be indicative of measurement signals generated by the multiple sensor devices 118. Rather than being directly functionally coupled or configured to communicate directly with the user device 160, as is the case in the example system 100 (**FIG. 1**), the wearable device 110 can be functionally coupled with the user device 160 via the network(s) 140.

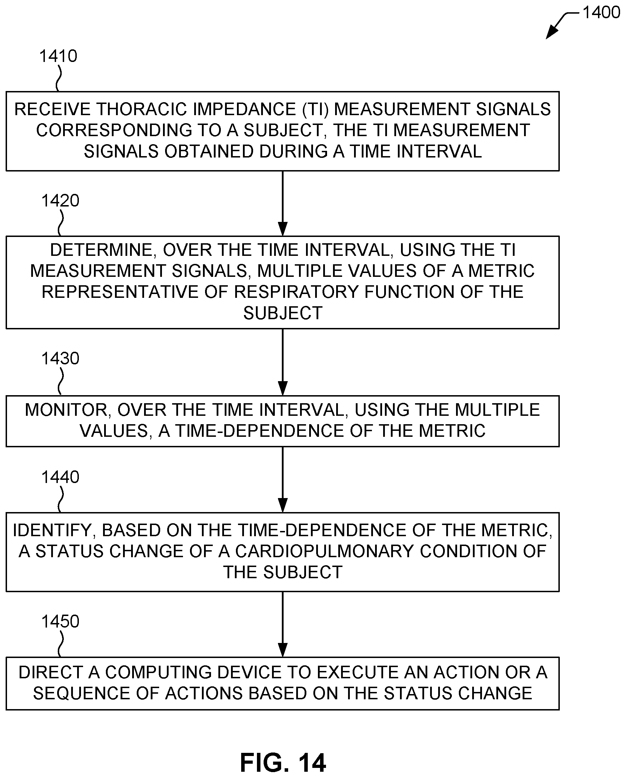

In view of the aspects described herein, example methods that may be implemented in accordance with this disclosure can be better appreciated with reference, for example, to the flowcharts in **FIGS. 14-17.** For the sake of simplicity of explanation, the example methods disclosed herein are presented and described as a series of blocks (with each block representing an action or an operation in a method, for example). However, the example methods are not limited by the order of blocks and associated actions or operations, as some blocks may occur in different orders and/or concurrently with other blocks from those that are shown and described herein. Further, not all illustrated blocks, and associated action(s), may be required to implement an example method in accordance with one or more aspects of the disclosure. Two or more of the example methods (and any other methods disclosed herein) may be implemented in combination with each other. It is noted that the example methods (and any other methods disclosed herein) may be alternatively represented as a series of interrelated states or events, such as in a state diagram.

**FIG. 14** is a flowchart of an example of a method for determining a status change of a cardiopulmonary condition, in accordance with one or more aspects of this disclosure. A computing device or a system of computing devices can implement the example method 1400 in its entirety or in part. To that end, each one of the computing devices includes computing resources that may implement at least one of the blocks included in the example method 1400 and other methods described herein. The computing resources comprise, for example, central processing units (CPUs), graphics processing units (GPUs), tensor processing units (TPUs), other types of processors, memory, disk space, incoming bandwidth, and/or outgoing bandwidth, interface(s) (such as I/O interfaces or APIs, or both); controller devices(s); power supplies; a combination of the foregoing; and/or similar resources. The computing device and the computing system can include multiple modules (e.g. the group of modules 156 or the software application described herein), and can implement the example method 1400 by executing one or more instances of such modules. To execute an instance of such modules (or other program code) one or more processors, individually or in combination, can execute the modules (or other program code), with the processor(s) being part of the computing resources mentioned above. In one example, the computing system is or includes the processing platform 150.

At block 1410, the computing system can receive thoracic impedance (TI) measurement signals corresponding to a subject. The TI measurement signals are time-dependent signals. For purposes of illustration, in aspects of this disclosure, a time-dependent signal may be a time series of values of a signal, where the signal has a value at one time (or instant) and another value at another time (or another instant). The value of the signal at the one time (or instant) may be same as or different from the other value at the other time (or other instant). A wearable device mounted on the torso of the subject (e.g., a chest of the subject) obtains the TI measurement signals during a time interval. In some cases, the computing system can thus receive or otherwise access the TI measurement signals from the wearable device (directly or indirectly). In other cases, the computing system can receive or otherwise access the TI measurement signals from another type of computing device that has access to the TI measurement signals and/or contains the TI measurement signals. The time interval includes an analysis period that spans two or more test periods. As is described herein, each test period can span 24 hours, in some cases. The TI measurement signals are obtained via an electric sensor device that is configured to measure impedance and is electrically coupled with electrodes in contact with respective sections of skin of the subject. The TI measurement signals can be obtained in a 4-electrode arrangement. Thus, the electric sensor is coupled with at least four electrodes. In one example, the wearable device is the wearable device 110 (**FIG. 1**), and the electric sensor device is included in the group of electric sensor devices 120a (**FIG. 1**) and the at least four electrodes are part of the multiple electrodes 114.

At block 1420, the computing system can determine, over the time interval, using the TI measurement signals, multiple values of a metric representative of respiratory function of the subject. The metric can be a direct metric or an aggregate metric. In one implementation, the metric is ratio of exhalation time and inhalation time of the subject. As is described herein, such a ratio is denoted by $\rho_{EI}$ (a real number in arbitrary units). In another implementation, the metric is magnitude of TI at peak in a pulmonary respiration cycle.

In implementations where the metric representative of respiratory function is $\rho_{EI}$, determining the multiple values of the metric includes operating on the TI measurement signals to generate pulmonary ventilation signals (also referred to as respiration signals) corresponding to the subject during the time interval. That is, in some implementations, the example method 1400 includes generating, using the TI measurement signals, respiration signals corresponding to the subject during the time interval. The computing system can then use the respiration signals to evaluate the metric for each measurement period included in the time interval. Hence, each one of the multiple values of the metric corresponds to a measurement period, as is described herein. In other words, in some implementations, the computing system can determine, over the time interval, using the respiration signals, multiple values of ratio of exhalation time and inhalation time of the subject.

At block 1430, the computing system can monitor, over the time interval, using the multiple values, a time dependence of the metric. In some cases, the monitoring can include determining that the time-dependence is indicative of an increase of the metric (e.g., ratio of exhalation time and inhalation time of the subject). In other cases, the monitoring can include determining that the time-dependence is indicative of a decrease of the metric (e.g., ratio of exhalation time and inhalation time of the subject). In yet other cases, the monitoring can include determining that the time-dependence is indicative of a nearly constant metric.

At block 1440, the computing system can identify, based on the time-dependence, a status change of a cardiopulmonary condition of the subject. In an implementation where the metric is the ratio of exhalation time and inhalation time of the subject, a time-dependence that is indicative of an increase of $\rho_{EI}$ can be indicative of an increase in airway obstruction and/or a worsened emphysematic condition and, thus, an exacerbation in the cardiopulmonary condition of the subject. In such an implementation, a time-dependence that is indicative of a decrease of $\rho_{EI}$ can be indicative of a decrease in airway restriction and, thus, an improvement in of the cardiopulmonary condition. Further, in such an implementation, a determination that the time-dependence of $\rho_{EI}$ is nearly constant can establish that the cardiopulmonary condition has remained unchanged. In case of a determination that the time-dependence of $\rho_{EI}$ is nearly constant and equal to approximately 1 (one), the status change can correspond to a CHF decompensation.

At block 1450, the computing system can direct or otherwise cause a computing device to execute an action or a sequence of actions based on the status change. In some cases, the computing device may be a user device. In addition, or in other cases, the computing device may be a clinician device or another device within a hospital system.

In some cases, as part of the example method 1400, the computing system can direct or otherwise cause, based on the status change, the wearable device to perform a measurement cycle of thoracic impedance of the subject. As is described herein, the wearable device may be a type of computing device.

In addition, or in other cases, also as part of the example method 1400, the computing system can prompt, based on the status change, the subject to cause the wearable device to perform a measurement of thoracic impedance of the subject over a measurement period.

Although the example method 1400 is described as being implemented by a computing system, such as the processing platform 150 (**FIG. 1**), the disclosure is not limited in that respect. Indeed, a computing device in accordance with aspects of this disclosure, such as the user device 160 or the computing device 170, also can implement the example method 1500.

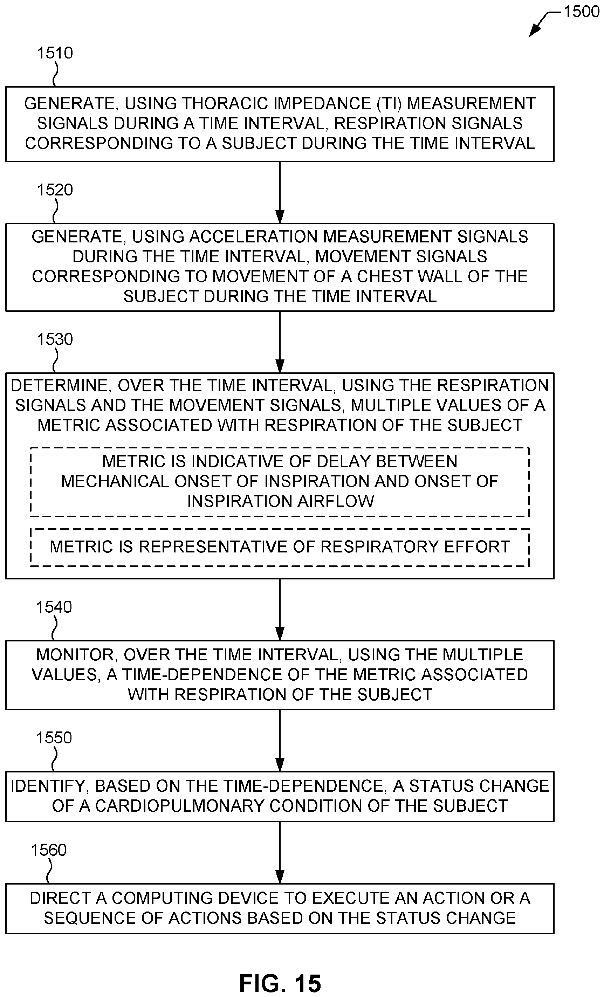

**FIG. 15** is a flowchart of an example of a method for determining a status change of a cardiopulmonary condition of a subject, in accordance with one or more aspects of this disclosure. The example method 1500 determines the status change of the cardiopulmonary condition by assessing respiratory effort of the subject. A computing device or a system of computing devices can implement the example method 1500 in its entirety or in part. To that end, each one of the computing devices includes computing resources that may implement at least one of the blocks included in the example method 1500 and other methods described herein. The computing resources include, for example, CPUs, graphics processing units GPUs, TPUs, memory, disk space, incoming bandwidth, and/or outgoing bandwidth, interface(s) (such as I/O interfaces or APIs, or both); controller devices(s); power supplies; a combination of the foregoing; and/or similar resources. The computing device and the computing system can include, for example, multiple modules (e.g., the group of modules 156 or the software application described herein) and can implement the example method 1500 by executing one or more instances of such modules. To execute an instance of such modules (or other program code) one or more processors, individually or in combination, can execute the modules (or other program code), with the processor(s) being part of the computing resources mentioned above. The computing system that implements the example method 1400 (**FIG. 14**) also can implement the example method 1500. In one example, the computing system is or includes the processing platform 150.

At block 1510, the computing system can generate, using thoracic impedance (TI) measurement signals during a time interval, respiration signals corresponding to a subject during the time interval. As is described herein, the TI measurement signals are time-dependent signals. Thus, the respiration signals also are time-dependent signals. A wearable device mounted on the torso of the subject can obtain the TI measurement signals during a time interval. The time interval includes an analysis period that spans two or more test periods. In some cases, as is described herein, each test period can span 24 hours. As is also described herein, the TI measurement signals can be obtained via an electric sensor device that is configured to measure impedance and is electrically coupled with electrodes in contact with respective areas of skin of the subject at respective skin locations on the torso of the subject (e.g., chest of the subject; **FIG. 3,** for example). The TI measurement signals can be obtained in a 4-electrode arrangement. Thus, in some cases, the electric sensor is coupled with at least four electrodes. In one example, the wearable device is the wearable device 110 (**FIG. 1**), and the electric sensor device is included in the group of electric sensor devices 120a (FIG. 1) and the at least four electrodes are part of the multiple electrodes 114.

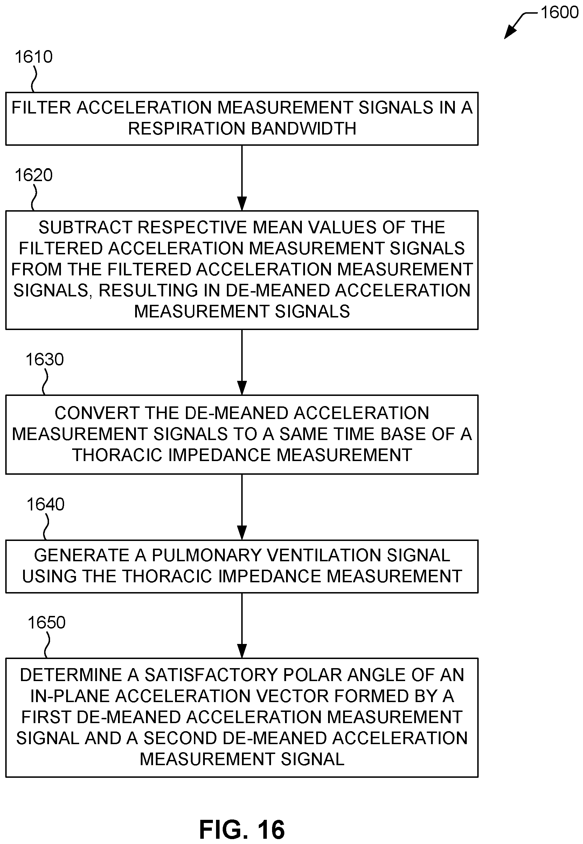

At block 1520, the computing system can generate, using acceleration measurement signals during the time interval, movement signals corresponding to movement of a chest wall of the subject during the time interval. The computing system can generate the movement signals by implementing the example method 1600 shown in **FIG. 16** and described hereinafter. As is described herein, the acceleration measurement signals are time-dependent signals. Thus, the movement signals also are time-dependent signals. The wearable device that obtains the TI measurement signals also can obtain that acceleration measurement signals. As is also described herein, the acceleration signals can be obtained via an inertial sensor (a three-axis accelerometer, for example) that is configured to measure direction and/or magnitude of an acceleration vector corresponding to the movement of the chest wall of the subject. Thus, the acceleration measurement signals can include first acceleration measurement signals corresponding to a component of the acceleration vector along a first axis (e.g., $a_x$), second acceleration measurement signals corresponding to another component of the acceleration vector along a second axis (e.g., $a_y$), and third acceleration measurement signals corresponding to yet another component of the acceleration vector along a third axis (e.g., $a_z$). The first axis, the second axis, and the third axis are mutually orthogonal. In cases where the wearable device is the wearable device 110 (**FIG. 1**), the inertial sensor device may be included in the group of inertial sensor devices 120c.

At block 1530, the computing system can determine, over the time interval, using the respiration signals and the movement signals, multiple values of a metric associated with respiration of the subject. For instance, the metric may be associated with respiratory function of the subject. In some cases, the metric is indicative of delay between mechanical onset of inspiration and onset of inspiration airflow. In other cases, the metric is representative of respiratory effort of the subject, with the metric being indicative of an amount of energy that is present in the movement signals relative to another amount of energy that is present in the respiration signals. An example metric that is representative of the respiratory effort of the subject is defined as a ratio of a variance of the movement signals and a variance of the respiration signals. The computing system can determine such a ratio in accordance with Eq. (1) or Eq. (2) hereinbefore.

In some cases, to determine the onset of inspiration airflow, using the respiration signals, the computing system can determine, by applying a zero-crossing processing to the respiration signals, a time instant corresponding to air flow exceeding a threshold amount. In addition, the computing system can then configure the time instant as the onset of inspiration airflow.

In addition, or in other cases, to the determine the onset of inspiration airflow, using the respiration signals, the computing system can determine, over a second time interval, airflow signals based on a first order derivative with respect to time of the respiration signals. In addition, the computing system can then determine a peak of the airflow signals during the second time interval, and can identify a time instant corresponding to the peak. Further, the computing system can configure the time instant as the onset of inspiration airflow.

With respect to mechanical onset of inspiration, the computing system can determine a trough of the movement signals during the second time interval. The computing system can then determine a peak of the movement signals during the second time interval. The computing system can then identify a second time instant corresponding to a value of the movement signals that exceeds a second value of the movement signals at the trough by a defined amount. The defined amount can be a defined fraction of a third value of the movement signals at the peak. The computing system can then configure the second time instant as the mechanical onset of inspiration.

At block 1540, the computing system can monitor, over the time interval, using the multiple values, a time-dependence of the metric associated with respiration of the subject.

At block 1550, the computing system can identify, based on the time-dependence, a status change of a cardiopulmonary condition of the subject. The computing system can identify the status change in accordance with aspects described herein.

At block 1560, the computing system can direct or otherwise cause a computing device to execute an action or a sequence of actions based on the status change. In some cases, the computing device may be a user device. In addition, or in other cases, the computing device may be a clinician device or another device within a hospital system.

Although the example method 1500 is described as being implemented by a computing system, such as the processing platform 150 (**FIG. 1**), the disclosure is not limited in that respect. Indeed, a computing device in accordance with aspects of this disclosure, such as the user device 160 or the computing device 170, also can implement the example method 1500.

**FIG. 16** is a flowchart of an example of a method for generating a movement signal corresponding to movement of the chest wall of a subject, in accordance with one or more aspects of this disclosure. As is described herein, in some cases, the computing system that implements the example method 1500 (**FIG. 15**) also can implement the example method 1600. At block 1610, the computing system can filter acceleration measurement signals in a respiration bandwidth (also referred to as breathing bandwidth), using a low-pass filter, resulting in filtered acceleration signals. For example, the computing system can filter a first acceleration measurement signal (e.g., time series of values of $a_x$) using the low-pass filter, resulting in a first filtered acceleration signal. In addition, the computing system can filter a second acceleration measurement signal (e.g., time series of values of $a_y$) using the low-pass filter, resulting in a second filtered acceleration signal. In some cases, the first acceleration measurement signal is a time series of values of $a_x$, and the second acceleration measurement signal is a time series of values of $a_y$. In other words, the acceleration measurement signals that are relied on to generate the movement signals may be in-plane acceleration signals.

As is described herein, the low-pass filter may be configured to remove frequencies exceeding a cutoff frequency within the respiration bandwidth (or breathing bandwidth). In this disclosure, the respiration bandwidth is a range of frequencies that contains most or essentially all likely frequencies of pulmonary ventilation in a human (e.g., an adult human). The range of frequencies can extend, for example, from about 0.05 Hz to about 0.75 Hz. In some implementations, the cutoff frequency is 0.75 Hz.

At block 1620, the computing system can subtract respective mean values of the filtered acceleration signals from the filtered acceleration measurement signals. Subtracting such a mean value yields a de-meaned acceleration measurement signal. In one example, the computing system can subtract a mean value of the filtered first acceleration measurement signal from the filtered first acceleration measurement signal, resulting in a de-meaned first acceleration measurement signal. Additionally, the computing system can subtract a mean value of the filtered second acceleration measurement signal from the filtered second acceleration measurement signal, resulting in a de-meaned second acceleration measurement signal.

At block 1630, the computing system can convert the de-meaned acceleration measurement signal to the time base of a TI measurement signal corresponding to the subject. Such a conversion permits directly comparing the de-meaned acceleration measurement signal and the TI measurement signal (or a signal derived therefrom) in the same time base. As is described herein, the wearable device that generates the acceleration measurement signals also can generate the TI measurement signal essentially concurrently with the generation of the acceleration measurement signals.

At block 1640, the computing system can generate a pulmonary ventilation signal for the subject using the TI measurement signal. The pulmonary ventilation signal is generated in accordance with aspects described herein. An example of the pulmonary ventilation signal is shown in **FIG. 5.**

At block 1650, the computing system can determine a satisfactory polar angle $\varphi_0$ of the in-plane acceleration vector formed by the first de-meaned acceleration measurement signal and the second de-meaned acceleration measurement signal. That is, as is described herein, the in-plane acceleration vector is a linear combination of the first and second de-meaned inertial measurement signals: $\Delta a_x \cos(\varphi) + \Delta a_y \sin(\varphi)$, where $\Delta a_x$ and $\Delta a_y$ represent, respectively the first and second de-meaned inertial measurement signals (or vice versa). The computing system can determine $\varphi_0$ by iteratively varying $\varphi$ and determining a correlation between $\Delta a_x \cos(\varphi) + \Delta a_y \sin(\varphi)$ and the pulmonary ventilation signal until the correlation is maximized or otherwise attains a satisfactory value. The satisfactory value may be configurable. Upon termination of such an iterative process, $\varphi_0$ is determined or otherwise identified as the value of $\varphi$ at the last iteration.

The computing system can then configure the movement signal representative of movement the chest wall of the subject as the linear combination of the first and second de-meaned inertial measurement signals that results from setting $\varphi$ to a value $\varphi_0$ that yields the maximal or otherwise satisfactory correlation. That is, the movement signal is configured as $\Delta a_x \cos(\varphi_0) + \Delta a_y \sin(\varphi_0)$.

Although the example method 1600 is described as being implemented by a computing system, such as the processing platform 150 **(FIG. 1),** the disclosure is not limited in that respect. Indeed, a computing device in accordance with aspects of this disclosure, such as the user device 160 or the computing device 170, also can implement the example method 1600.

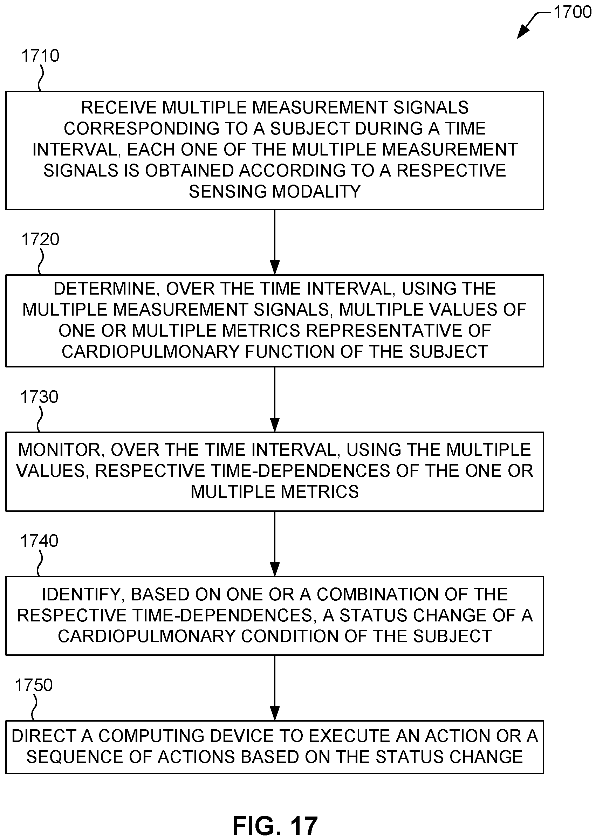

**FIG. 17** is a flowchart of an example of a method for determining a status change of a cardiopulmonary condition, in accordance with one or more embodiments of this disclosure. A computing device or a system of computing devices can implement the example method 1700 in its entirety or in part. To that end, each one of the computing devices includes computing resources that may implement at least one of the blocks included in the example method 1800 and other methods described herein. The computing resources comprise, for example, CPUs, graphics processing units CPUs, TPUs, memory, disk space, incoming bandwidth, and/or outgoing bandwidth, interface(s) (such as I/O interfaces or APIs, or both); controller devices(s); power supplies; a combination of the foregoing; and/or similar resources. The computing device and the computing system can include multiple modules (e.g., the group of modules 156 or the software application described herein) and can implement the example method 1700 by executing one or more instances of such modules. To execute an instance of such modules (or other program code) one or more processors, individually or in combination, can execute the modules (or other program code), with the processor(s) being part of the computing resources mentioned above. The computing system that implements the example method 1400 **(FIG. 14),** the example method 1500 **(FIG. 15),** the example method 1600 **(FIG. 16),** or two or more of those methods, also can implement the example method 1700. In one example, the computing system is or includes the processing platform 150.

At block 1710, the computing system can receive multiple measurement signals corresponding to a subject during a time interval. The time interval includes an analysis period that spans two or more test periods. As is described herein, each test period can span 24 hours, in some cases. Each one of the multiple measurement signals is obtained according to a respective sensing modality during a time interval. A wearable device (e.g., wearable device 110 **(FIG. 1)**) mounted on the torso of the subject obtains the multiple measurement signals during the time interval. The wearable device includes multiple sensor devices (e.g., sensor devices 118 **(FIG. 1)**). The multiple sensor devices include groups of sensor devices, each group having one or more Sensor devices. Each group of sensor devices is configured to probe a particular physical quantity of the subject, where the particular physical quantity of the subject is associated with cardiopulmonary function of the subject. The probing of the particular physical quantity is referred to as a sensing modality. Accordingly, each group of sensor devices is configured to generate measurement signals according to a particular sensor modality. As is described herein, the multiple sensor devices can include one or a combination of (i) a group of electric sensor devices 120a, (ii) a group of acoustic sensor devices 120b, (iii) a group of inertial sensor devices 120c, (iv) or an oximeter device 120d.

At block 1720, the computing system can determine, over the time interval, using the multiple measurement signals, multiple values of one or multiple metrics representative of cardiopulmonary function of the subject. As is described herein, each one of the metric(s) can be either a direct metric or an aggregate metric.

At block 1730, the computing system can monitor, over the time interval, using the multiple values, respective time-dependences of the one or multiple metrics. A time-dependence of a metric can be referred to as trend of the metric.

At block 1740, the computing system can identify, based on one or a combination of the respective time-dependences (or respective trends), a status change of a cardiopulmonary condition of the subject. The status change can be identified in accordance with aspects described herein.

At block 1750, the computing system can direct or otherwise cause a computing device to execute an action or a sequence of actions based on the status change. In some cases, the computing device may be a user device. In addition, or in other cases, the computing device may be a clinician device or another device within a hospital system.

Although the example method 1700 is described as being implemented by a computing system, such as the processing platform 150 **(FIG. 1),** the disclosure is not limited in that respect. Indeed, a computing device in accordance with aspects of this disclosure, such as the user device 160 or the computing device 170, also can implement the example method 1700.

Numerous example embodiments emerge from the foregoing detailed description and annexed drawings. The example embodiments include the following:

Example 1. A system, comprising: a wearable device configured to be mounted on a torso of a subject, the wearable device comprising multiple electrodes and multiple sensor devices, wherein each one of the multiple electrodes is configured to contact a respective skin area at a respective skin location on the torso of the subject, and wherein the multiple sensor devices include: an electric sensor device functionally coupled with the multiple electrodes and configured to generate first measurement signals during a measurement period; and a movement sensor device configured to generate second measurement signals during the measurement period; and a computing device configured to communicate with the wearable device and configured to: receive the first measurement signals and the second measurement signals; and determine, using a combination of the first measurement signals and the second measurement signals, a value of a metric or respective values in a combination of metrics, wherein each one of the metric and the combination of metrics is indicative of status of a cardiopulmonary condition of the subject.

Example 2. The system of Example 1, wherein the computing device is further configured to identify, based on a change of the value relative to a past value of the metric, a status change of the cardiopulmonary condition.

Example 3. The system of Example 1 or Example 2, wherein the computing device is further configured to identify, based on individual changes of the respective values relative to respective past values of the metrics in the combination of metrics, a status change of the cardiopulmonary condition.

Example 4. The system of any of the preceding Examples, wherein the movement sensor device comprises an inertial sensor device.

Example 5. The system of any of the preceding Examples, wherein the wearable device further comprises a haptic device and is configured to: determine that placement of the wearable device on the torso of the subject fails to satisfy a placement rule; direct the haptic device to cause vibration of the wearable device; determine that an updated placement of the wearable device on the torso of the subject satisfies the placement rule; and direct the haptic device to cease vibration of the wearable device.

Example 6. The system of any of the preceding Examples, wherein the computing device is further configured to provide an indication of an action to be executed by the subject prior to the measurement period, the action comprising performing one or more movements to stress a cardiopulmonary system of the subject.

Example 7. The system of any of the preceding Examples, wherein the computing device is further configured to: obtain multiple second values of the metric for respective second measurement periods within an analysis period; monitor, over the analysis period, using the value and the multiple second values, a time-dependence of the metric; and identify, based on the time-dependence, a status change of the cardiopulmonary condition.

Example 8. The system of any of the preceding Examples, wherein the computing device is further configured to: obtain multiple second respective values of the combination of metrics for respective second measurement periods within an analysis period; monitor, over the analysis period, using the respective values and the multiple second respective values, respective time-dependences of the metrics in the combination of metrics; and identify, based on the respective time-dependences, a status change of the cardiopulmonary condition.

Example 9. The system of any of the preceding Examples, wherein the measurement period has a duration within a range from about 25 seconds to about 120 seconds.

Example 10. The system of any of the preceding Examples, wherein the measurement period has a duration within a range from about 25 seconds to about five days.

Example 11. The system of any of the preceding Examples, wherein the analysis period has a duration from at least one hour, with the duration being greater than another duration of the measurement period.

Example 12. The system of any of the preceding Examples, wherein the computing device is further configured to cause, based on the status change, the wearable device to generate additional measurement signals.

Example 13. The system of any of the preceding Examples, wherein the computing device is further configured to prompt, based on a first value of a particular metric or respective second values in a particular combination of metrics, one or more of the subject, a clinician, or a caregiver to cause the wearable device to generate additional measurement signals.

Example 14. The system of any of the preceding Examples, wherein the computing device is further configured to provide an indication of an action to be executed by the subject in response to at least one of a first value of a particular metric, a first value of a combination of metrics, or the status change.

Example 15. The system of any of the preceding Examples, wherein to provide the indication of the action to be executed by the subject, the computing device is configured to cause presentation of a message directing the subject to proceed to an emergency room, a second message directing the subject to contact a primary physician, or a third message directing the subject to contact an aid.

Example 16. The system of any of the preceding Examples, wherein to provide the indication of the action to be executed by the subject, the computing device is configured to cause transmission of an electronic communication to a clinician device or a second electronic communication to a caregiver device, with the electronic communication and the second electronic communication including the indication.

Example 17. The system of any of the preceding Examples, wherein to provide the indication of the action to be executed by the subject, the computing device is configured to present a message directing one or more of the subject, a clinician, or a caregiver to adjust placement of the wearable device on the torso.

Example 18. The system of any of the preceding Examples, wherein the computing device is further configured to execute program code and, in response, present a patient reported outcome (PRO) questionnaire associated with the cardiopulmonary condition.

Example 19. The system of any of the preceding Examples, wherein the multiple sensor devices further include an acoustic sensor device configured to generate third measurement signals during the measurement period.

Example 20. The system of any of the preceding Examples, wherein the computing device is further configured to: receive the first measurement signals, the second measurement signals, and the third measurement signals; and determine, using a combination of the first measurement signals, the second measurement signals, and the third measurement signals, another value of the metric or respective other values in the combination of metrics.

Example 21. The system of any of the preceding Examples, wherein the multiple sensor devices further include an optical device configured to generate fourth measurement signals during the measurement period.

Example 22. The system of any of the preceding Examples, wherein the optical device comprises a pulse oximeter.

Example 23. The system of any of the preceding Examples, wherein the computing device is further configured to: receive the first measurement signals, the second measurement signals, the third measurement signals, and the fourth measurement signals; and determine, using a combination of the first measurement signals, the second measurement signals, the third measurement signals, and the fourth measurement signals, yet another value of the metric or respective yet other values in the combination of metrics.

Example 24. The system of any of the preceding Examples, wherein the computing device is further configured to: determine that the fourth measurement signals are indicative of an oxygen saturation level that is less than a threshold level; determine that a status change of the cardiopulmonary condition is indicative of exacerbation of the cardiopulmonary condition; and present a message directing the subject to proceed to an emergency room, another message directing the subject to contact a primary physician, or yet another message directing the subject to contact an aid.

Example 25. A method, comprising: generating, by one or more processors, individually or in combination, using thoracic impedance (TI) measurement signals during a time interval, respiration signals corresponding to a subject, wherein the TI measurement signals are time-dependent; generating, by the one or more processors, individually or in combination, using acceleration measurement signals during the time interval, movement signals corresponding to movement of a chest wall of the subject, wherein the acceleration measurement signals are time-dependent; determining, by the one or more processors, individually or in combination, over the time interval, using the respiration signals and the movement signals, multiple values of a metric associated with respiration of the subject; monitoring, by the one or more processors, individually or in combination, over the time interval, using the multiple values, a time-dependence of the metric; and identifying, by the one or more processors, individually or in combination, based on the time-dependence, a status change of a cardiopulmonary condition of the subject.

Example 26. The method of Example 25, wherein the time interval comprises an analysis period that spans at least one test period, with each of the at least one test period spanning multiple measurement periods.

Example 27. The method of Example 25 or Example 26, wherein the TI measurement signals are obtained by a wearable device mounted on the chest of the subject, and wherein the acceleration measurement signals are obtained by the wearable device.

Example 28. The method of any of the preceding Examples, wherein the metric is indicative of delay between mechanical onset of inspiration and onset of inspiration airflow.

Example 29. The method of any of the preceding Examples, further comprising determining, by the one or more processors, individually or in combination, using the respiration signals, the onset of inspiration airflow.

Example 30. The method of any of the preceding Examples, wherein the determining the onset of inspiration airflow comprises: determining, by applying a zero-crossing processing to the respiration signals, a time instant corresponding to air flow exceeding a threshold amount; and configuring the time instant as the onset of inspiration airflow.

Example 31. The method of any of the preceding Examples, wherein the determining the onset of inspiration airflow comprises: determining, over a second time interval, air flow signals based on a first order derivative with respect to time of the respiration signals; determining a peak of the air flow signals during the second time interval; identifying a time instant corresponding to the peak; and configuring the time instant as the onset of inspiration airflow.

Example 32. The method of any of the preceding Examples, further comprising: determining, by the one or more processors, individually or in combination, a trough of the movement signals during the second time interval; determining, by the one or more processors, individually or in combination, a peak of the movement signals during the second time interval; identifying, by the one or more processors, individually or in combination, a second time instant corresponding to a value of the movement signals that exceeds a second value of the movement signals at the trough by a defined amount, wherein the defined amount is a defined fraction of a third value of the movement signals at the peak; and configuring, by the one or more processors, individually or in combination, the second time instant as the mechanical onset of inspiration.

Example 33. The method of any of the preceding Examples, wherein the metric is representative of respiratory effort of the subject, with the metric being indicative of an amount of energy present in the movement signals relative to a second amount of energy present in the respiration signals.

Example 34. The method of any of the preceding Examples, wherein the metric is defined as a ratio of a variance of the movement signals and a variance of the respiration signals.

Example 35. A computing system, comprising: at least one processor; and at least one memory device storing processor-executable instructions that, in response to execution by the at least one processor, individually or in combination, cause the computing system at least to: generate, using thoracic impedance (TI) measurement signals during a time interval, respiration signals corresponding to a subject, wherein the TI measurement signals are time-dependent; generate, using acceleration measurement signals during the time interval, movement signals corresponding to movement of a chest wall of the subject, wherein the acceleration measurement signals are time-dependent; determine, over the time interval, using the respiration signals and the movement signals, multiple values of a metric associated with respiration of the subject; monitor, over the time interval, using the multiple values, a time-dependence of the metric; and identify, based on the time-dependence, a status change of a cardiopulmonary condition of the subject.

Example 36. The computing system of Example 35, wherein the time interval comprises an analysis period that spans at least one test period, with each of the at least one test period spanning multiple measurement periods.

Example 37. The computing system of Example 35 or Example 36, wherein the TI measurement signals are obtained by a wearable device mounted on the chest of the subject, and wherein the acceleration measurement signals are obtained by the wearable device.

Example 38. The computing system of any of the preceding Examples, wherein the metric is indicative of delay between mechanical onset of inspiration and onset of inspiration airflow.

Example 39. The computing system of any of the preceding Examples, wherein the processor-executable instructions, in response to execution by the at least one processor, individually or in combination, further cause the computing system to determine, by the at least one processor, individually or in combination, using the respiration signals, the onset of inspiration airflow.

Example 40. The computing system of any of the preceding Examples, wherein to determine the onset of inspiration airflow, the processor-executable instructions, in response to execution by the at least one processor, individually or in combination, further cause the computing system at least to: determine, by applying a zero-crossing processing to the respiration signals, a time instant corresponding to air flow exceeding a threshold amount; and configure the time instant as the onset of inspiration airflow.

Example 41. The computing system of any of the preceding Examples, wherein to determine the onset of inspiration airflow the processor-executable instructions, in response to execution by the at least one processor, individually or in combination, further cause the computing system at least to: determine, over a second time interval, air flow signals based on a first order derivative with respect to time of the respiration signals; determine a peak of the air flow signals during the second time interval; identify a time instant corresponding to the peak; and configure the time instant as the onset of inspiration airflow.

Example 42. The computing system of any of the preceding Examples, wherein the processor-executable instructions, in response to execution by the at least one processor, individually or in combination, further cause the computing system at least to: determine a trough of the movement signals during the second time interval; determine a peak of the movement signals during the second time interval; identify a second time instant corresponding to a value of the movement signals that exceeds a second value of the movement signals at the trough by a defined amount, wherein the defined amount is a defined fraction of a third value of the movement signals at the peak; and configure the second time instant as the mechanical onset of inspiration.

Example 43. The computing system of any of the preceding Examples, wherein the metric is representative of respiratory effort of the subject, with the metric being indicative of an amount of energy present in the movement signals relative to a second amount of energy present in the respiration signals.

Example 44. The computing system of any of the preceding Examples, wherein the metric is defined as a ratio of a variance of the movement signals and a variance of the respiration signals.

Example 45. A user device, comprising: at least one processor; and at least one memory device storing processor-executable instructions that, in response to execution by the at least one processor, individually or in combination, cause the user device at least to: generate, using thoracic impedance (TI) measurement signals during a time interval, respiration signals corresponding to a subject, wherein the TI measurement signals are time-dependent; generate, using acceleration measurement signals during the time interval, movement signals corresponding to movement of a chest wall of the subject, wherein the acceleration measurement signals are time-dependent; determine, over the time interval, using the respiration signals and the movement signals, multiple values of a metric associated with respiration of the subject; monitor, over the time interval, using the multiple values, a time-dependence of the metric; and identify, based on the time-dependence, a status change of a cardiopulmonary condition of the subject.

Example 46. The user device of Example 45, wherein the time interval comprises an analysis period that spans at least one test period, with each of the at least one test period spanning multiple measurement periods.

Example 47. The user device of Example 45 or Example 46, wherein the TI measurement signals are obtained by a wearable device mounted on the chest of the subject, and wherein the acceleration measurement signals are obtained by the wearable device.

Example 48. The user device of any of the preceding Examples, wherein the metric is indicative of delay between mechanical onset of inspiration and onset of inspiration airflow.

Example 49. The user device of any of the preceding Examples, wherein the processor-executable instructions, in response to execution by the at least one processor, individually or in combination, further cause the user device to determine, by the at least one processor, individually or in combination, using the respiration signals, the onset of inspiration airflow.

Example 50. The user device of any of the preceding Examples, wherein to determine the onset of inspiration airflow, the processor-executable instructions, in response to execution by the at least one processor, individually or in combination, further cause the user device at least to: determine, by applying a zero-crossing processing to the respiration signals, a time instant corresponding to air flow exceeding a threshold amount; and configure the time instant as the onset of inspiration airflow.

Example 51. The user device of any of the preceding Examples, wherein to determine the onset of inspiration airflow the processor-executable instructions, in response to execution by the at least one processor, individually or in combination, further cause the user device at least to: determine, over a second time interval, air flow signals based on a first order derivative with respect to time of the respiration signals; determine a peak of the air flow signals during the second time interval; identify a time instant corresponding to the peak; and configure the time instant as the onset of inspiration airflow.

Example 52. The user device of any of the preceding Examples, wherein the processor-executable instructions, in response to execution by the at least one processor, individually or in combination, further cause the user device at least to: determine a trough of the movement signals during the second time interval; determine a peak of the movement signals during the second time interval; identify a second time instant corresponding to a value of the movement signals that exceeds a second value of the movement signals at the trough by a defined amount, wherein the defined amount is a defined fraction of a third value of the movement signals at the peak; and configure the second time instant as the mechanical onset of inspiration.

Example 53. The user device of any of the preceding Examples, wherein the metric is representative of respiratory effort of the subject, with the metric being indicative of an amount of energy present in the movement signals relative to a second amount of energy present in the respiration signals.

Example 54. The user device of any of the preceding Examples, wherein the metric is defined as a ratio of a variance of the movement signals and a variance of the respiration signals.

Example 55. A method, comprising: receiving, by one or more processors, individually or in combination, thoracic impedance (TI) measurement signals corresponding to a subject, the TI measurement signals being time-dependent and obtained during a time interval; generating, by the one or more processors, individually or in combination, using the TI measurement signals, respiration signals corresponding to the subject during the time interval; determining, by the one or more processors, individually or in combination, over the time interval, using the respiration signals, multiple values of a ratio of an exhalation time and an inhalation time of the subject; monitoring, by the one or more processors, individually or in combination, over the time interval, using the multiple values, a time-dependence of the ratio; and identifying, by the one or more processors, individually or in combination, based on the time-dependence, a status change of a cardiopulmonary condition of the subject.

Example 56. The method of Example 55, further comprising causing by the one or more processors, individually or in combination, a computing device to present one or more markings indicative of the status change.

Example 57. The method of Example 55 or Example 56, wherein the cardiopulmonary condition includes at least one of airway constriction or emphysema, the method further comprising, determining, by the one or more processors, individually or in combination, that the time-dependence is indicative of an increase of the ratio of the exhalation time and the inhalation time of the subject; and identifying, by the one or more processors, individually or in combination, the status change as an exacerbation of the cardiopulmonary condition.

Example 58. The method of any of the preceding Examples, wherein the cardiopulmonary condition includes at least one of airway constriction or emphysema, the method further comprising, determining, by the one or more processors, individually or in combination, that the time-dependence is indicative of a decrease of the ratio of the exhalation time and the inhalation time of the subject; and identifying, by the one or more processors, individually or in combination, the status change as an improvement of the cardiopulmonary condition.

Example 59. The method of any of the preceding Examples, wherein the TI measurement signals are received from a wearable device mounted on a torso of the subject, the method further comprising causing, by the one or more processors, individually or in combination, based on the status change, the wearable device to perform a measurement cycle of thoracic impedance of the subject.

Example 60. The method of any of the preceding Examples, wherein the TI measurement signals are received from a wearable device mounted on a torso of the subject, the method further comprising prompting, by the one or more processors, individually or in combination, based on the status change, the subject to cause the wearable device to perform a measurement of thoracic impedance of the subject over a measurement period.

Example 61. The method of any of the preceding Examples, wherein the generating, using the TI measurement signals, the respiration signals comprises, filtering the TI measurement signals using a low-pass filter configured to remove frequencies exceeding a cutoff frequency within a respiration bandwidth; subtracting a mean value of the filtered TI measurement signals from the TI measurement signals, resulting in de-meaned TI measurement signals; and subtracting a moving average of the de-meaned TI measurement signals from the de-meaned TI measurement signals, resulting in the respiration signals.

Example 62. A computing system, comprising: at least one processor; and at least one memory device storing processor-executable instructions that, in response to execution by the at least one processor, individually or in combination, cause the computing system at least to: receive thoracic impedance (TI) measurement signals corresponding to a subject, the TI measurement signals being time-dependent and obtained during a time interval; generate, using the TI measurement signals, respiration signals corresponding to the subject during the time interval;

determine, over the time interval, using the respiration signals, multiple values of a ratio of an exhalation time and an inhalation time of the subject; monitor, over the time interval, using the multiple values, a time-dependence of the ratio; and identify, based on the time-dependence, a status change of a cardiopulmonary condition of the subject.

Example 63. The computing system of Example 62, wherein the processor-executable instructions, in response to execution by the at least one processor, individually or in combination, further cause the computing system to cause a computing device to present one or more markings indicative of the status change.

Example 64. The computing system of Example 62 of Example 63, wherein the cardiopulmonary condition includes at least one of airway constriction or emphysema, and wherein the processor-executable instructions, in response to execution by the at least one processor, individually or in combination, further cause the computing system to, determine that the time-dependence is indicative of an increase of the ratio of the exhalation time and the inhalation time of the subject; and identify the status change as an exacerbation of the cardiopulmonary condition.

Example 65. The computing system of any of the preceding Examples, wherein the cardiopulmonary condition includes at least one of airway constriction or emphysema, and wherein the processor-executable instructions, in response to execution by the at least one processor, individually or in combination, further cause the computing system to, determine that the time-dependence is indicative of a decrease of the ratio of the exhalation time and the inhalation time of the subject; and identify the status change as an improvement of the cardiopulmonary condition.

Example 66. The computing system of any of the preceding Examples, wherein the TI measurement signals are received from a wearable device mounted on a torso of the subject, and wherein the processor-executable instructions, in response to execution by the at least one processor, individually or in combination, further cause the computing system to cause, based on the status change, the wearable device to perform a measurement cycle of thoracic impedance of the subject.

Example 67. The computing system of any of the preceding Examples, wherein the TI measurement signals are received from a wearable device mounted on a torso of the subject, and wherein the processor-executable instructions, in response to execution by the at least one processor, individually or in combination, further cause the computing system to prompt, based on the status change, the subject to cause the wearable device to perform a measurement of thoracic impedance of the subject over a measurement period.

Example 68. The computing system of any of the preceding Examples, wherein to generate, using the TI measurement signals, the respiration signals, the processor-executable instructions, in response to execution by the at least one processor, individually or in combination, further cause the computing system at least to, filter the TI measurement signals using a low-pass filter configured to remove frequencies exceeding a cutoff frequency within a respiration bandwidth; subtract a mean value of the filtered TI measurement signals from the TI measurement signals, resulting in de-meaned TI measurement signals; and subtract a moving average of the de-meaned TI measurement signals from the de-meaned TI measurement signals, resulting in the respiration signals.

Example 69. A user device, comprising: at least one processor; and at least one memory device storing processor-executable instructions that, in response to execution by the at least one processor, individually or in combination, cause the user device at least to: receive thoracic impedance (TI) measurement signals corresponding to a subject, the TI measurement signals being time-dependent and obtained during a time interval; generate, using the TI measurement signals, respiration signals corresponding to the subject during the time interval; determine, over the time interval, using the respiration signals, multiple values of a ratio of an exhalation time and an inhalation time of the subject; monitor, over the time interval, using the multiple values, a time-dependence of the ratio; and identify, based on the time-dependence, a status change of a cardiopulmonary condition of the subject.

Example 70. The user device of Example 69, wherein the processor-executable instructions, in response to execution by the at least one processor, individually or in combination, further cause the user device to cause a computing device to present one or more markings indicative of the status change.

Example 71. The user device of Example 69 or Example 70, wherein the cardiopulmonary condition includes at least one of airway constriction or emphysema, and wherein the processor-executable instructions, in response to execution by the at least one processor, individually or in combination, further cause the user device to, determine that the time-dependence is indicative of an increase of the ratio of the exhalation time and the inhalation time of the subject; and identify the status change as an exacerbation of the cardiopulmonary condition.

Example 72. The user device of any of the preceding Examples, wherein the cardiopulmonary condition includes at least one of airway constriction or emphysema, and wherein the processor-executable instructions, in response to execution by the at least one processor, individually or in combination, further cause the user device to, determine that the time-dependence is indicative of a decrease of the ratio of the exhalation time and the inhalation time of the subject; and identify the status change as an improvement of the cardiopulmonary condition.

Example 73. The user device of any of the preceding Examples, wherein the TI measurement signals are received from a wearable device mounted on a torso of the subject, and wherein the processor-executable instructions, in response to execution by the at least one processor, individually or in combination, further cause the user device to cause, based on the status change, the wearable device to perform a measurement cycle of thoracic impedance of the subject.

Example 74. The user device of any of the preceding Examples, wherein the TI measurement signals are received from a wearable device mounted on a torso of the subject, and wherein the processor-executable instructions, in response to execution by the at least one processor, individually or in combination, further cause the user device to prompt, based on the status change, the subject to cause the wearable device to perform a measurement of thoracic impedance of the subject over a measurement period.

Example 75. The user device of any of the preceding Examples, wherein to generate, using the TI measurement signals, the respiration signals, the processor-executable instructions, in response to execution by the at least one processor, individually or in combination, further cause the user device at least to, filter the TI measurement signals using a low-pass filter configured to remove frequencies exceeding a cutoff frequency within a respiration bandwidth; subtract a mean value of the filtered TI measurement signals from the TI measurement signals, resulting in de-meaned TI measurement signals; and subtract a moving average of the de-meaned TI measurement signals from the de-meaned TI measurement signals, resulting in the respiration signals.

Various aspects of the disclosure may take the form of an entirely or partially hardware aspect, an entirely or partially software aspect, or a combination of software and hardware. Furthermore, as described herein, various aspects of the disclosure (e.g., systems and methods) may take the form of a computer program product comprising a computer-readable non-transitory storage medium having processor-accessible instructions (e.g., computer-readable and/or computer-executable instructions) such as computer software, encoded or otherwise embodied in such storage medium. Those instructions can be read or otherwise accessed and executed by one or more processors, individually or in combination, to perform or permit the performance of the operations described herein. The instructions can be provided in any suitable form, such as source code, compiled code, interpreted code, executable code, static code, dynamic code, assembler code, combinations of the foregoing, and the like. Any suitable computer-readable non-transitory storage medium may be utilized to form the computer program product. For instance, the computer-readable medium may include any tangible non-transitory medium for storing information in a form readable or otherwise accessible by one or more computers or processor(s) functionally coupled thereto. Non-transitory storage media can include read-only memory (ROM); random access memory (RAM); magnetic disk storage media; optical storage media; flash memory, and so forth.

Aspects of this disclosure are described herein with reference to block diagrams and flowchart illustrations of processor-implemented methods, systems, devices, apparatuses, and computer program products. It can be understood that each block of the block diagrams and flowchart illustrations, and combinations of blocks in the block diagrams and flowchart illustrations, respectively, can be implemented by processor-accessible instructions. Such instructions may include, for example, computer program instructions (e.g., processor-readable and/or processor-executable instructions). The processor-accessible instructions may be built (e.g., linked and compiled) and retained in processor-executable form in one or multiple memory devices or one or many other processor-accessible non-transitory storage media. These computer program instructions also can be stored in a processor-readable memory, where in response to execution by one or more processors, individually or in combination, the computer program instructions can direct a computer, a computing device, or other programmable data processing apparatus to function in a particular manner, such that the instructions stored in the processor-readable memory produce an article of manufacture including processor-accessible instructions (e.g., processor-readable instructions and/or processor-executable instructions) to implement the function specified in the flowchart blocks (individually or in a particular combination) or blocks in block diagrams (individually or in a particular combination). The computer program instructions can be loaded onto a computer, a computing device, or other programmable data processing apparatus to cause a series of operations to be performed on the computer or other programmable apparatus to produce a computer-implemented process. The series of operations may be performed in response to execution by one or more processor or other types of processing circuitry. Thus, such instructions that execute on the computer or other programmable apparatus provide operations for implementing the functions specified in the flowchart blocks (individually or in a particular combination) or blocks in block diagrams (individually or in a particular combination).

In some implementations, the processor-accessible instructions may be loaded or otherwise incorporated into a general purpose computer, a special purpose computer, or another programmable information processing apparatus to produce a particular machine, such that the operations or functions specified in the flowchart block or blocks can be implemented in response to execution at the computer or processing apparatus. More specifically, the loaded processor-accessible instructions may be accessed and executed by one or multiple processors, individually or in combination, or other types of processing circuitry. In response to execution, the loaded processor-accessible instructions provide the functionality described in connection with flowchart blocks (individually or in a particular combination) or blocks in block diagrams (individually or in a particular combination). Thus, such instructions which execute on a computer, a computing device, or other programmable data processing apparatus can create a means for implementing the functions specified in the flowchart blocks (individually or in a particular combination) or blocks in block diagrams (individually or in a particular combination).

Unless otherwise expressly stated, it is in no way intended that any protocol, procedure, process, or method set forth herein be construed as requiring that its acts or steps be performed in a specific order. Accordingly, where a process or method claim does not actually recite an order to be followed by its acts or steps or it is not otherwise specifically recited in the claims or descriptions of the subject disclosure that the steps are to be limited to a specific order, it is in no way intended that an order be inferred, in any respect. This holds for any possible non-express basis for interpretation, including: matters of logic with respect to the arrangement of steps or operational flow; plain meaning derived from grammatical organization or punctuation; the number or type of aspects described in the specification or annexed drawings; or the like.

As used in this disclosure, including the annexed drawings, the terms "component," "module," "interface," "system," and the like are intended to refer to a computer-related entity or an entity related to an apparatus with one or more specific functionalities. The entity can be either hardware, a combination of hardware and software, software, or software in execution. One or more of such entities are also referred to as "functional elements." As an example, a component can be a process running on a processor, a processor, an object, an executable, a thread of execution, a program, and/or a computer. For example, both an application running on a server or network controller, and the server or network controller can be a component. One or more components can reside within a process and/or thread of execution and a component can be localized on one computer and/or distributed between two or more computers. Also, these components can execute from various computer readable media having various data structures stored thereon. The components can communicate via local and/or remote processes such as in accordance with a signal having one or more data packets (e.g., data from one component interacting with another component in a local system, distributed system, and/or across a network such as the Internet with other systems via the signal). As another example, a component can be an apparatus with specific functionality provided by mechanical parts operated by electric or electronic circuitry, which parts can be controlled or otherwise operated by program code executed by a processor. As yet another example, a component can be an apparatus that provides specific functionality through electronic components without mechanical parts, the electronic components can include a processor to execute program code that provides, at least partially, the functionality of the electronic components. As still another example, interface(s) can include I/O components or Application Programming Interface (API) components. While the foregoing examples are directed to aspects of a component, the exemplified aspects or features also apply to a system, module, and similar.

In addition, the term "or" is intended to mean an inclusive "or" rather than an exclusive "or." That is, unless specified otherwise, or clear from context, "X employs A or B" is intended to mean any of the natural inclusive permutations. That is, if X employs A; X employs B; or X employs both A and B, then "X employs A or B" is satisfied under any of the foregoing instances. Moreover, articles "a" and "an" as used in this specification and annexed drawings should be construed to mean "one or more" unless specified otherwise or clear from context to be directed to a singular form.

In addition, the terms "example" and "such as" are utilized herein to mean serving as an instance or illustration. Any aspect or design described herein as an "example" or referred to in connection with a "such as" clause is not necessarily to be construed as preferred or advantageous over other aspects or designs described herein. Rather, use of the terms "example" or "such as" is intended to present concepts in a concrete fashion. The terms "first," "second," "third," and so forth, as used in the claims and description, unless otherwise clear by context, is for clarity only and doesn't necessarily indicate or imply any order in time or space.

The term "processor," as utilized in this disclosure, can refer to any computing processing unit or device comprising processing circuitry that can operate on data and/or signaling. A computing processing unit or device can include, for example, single-core processors; single-processors with software multithread execution capability; multi-core processors; multi-core processors with software multithread execution capability; multi-core processors with hardware multithread technology; parallel platforms; and parallel platforms with distributed shared memory. Additionally, a processor can include an integrated circuit, an application specific integrated circuit (ASIC), a digital signal processor (DSP), a field programmable gate array (FPGA), a programmable logic controller (PLC), a complex programmable logic device (CPLD), a discrete gate or transistor logic, discrete hardware components, or any combination thereof designed to perform the functions described herein. In some cases, processors can exploit nano-scale architectures, such as molecular and quantum-dot based transistors, switches and gates, in order to optimize space usage or enhance performance of user equipment. A processor may also be implemented as a combination of computing processing units. A processor may also be implemented as a combination of computing processing units. In addition, or as an alternative, a processor may be implemented as a virtual machine (or virtual processor) where a host device can provide a software environment in which the virtual processor shares computing resources of the host device with other virtual processors and/or components of the host device. The software environment may be referred to as a virtualized environment and permits the virtual processor to perform operations by executing processor executable instructions retained in a portion of the underlying computing resources. As is described herein, the computing resources may include, for example, an operating system (O/S), CPUs, memory, disk space, incoming bandwidth, and/or outgoing bandwidth.

In addition, terms such as "store," "data store," "data storage," "database," and substantially any other information storage component relevant to operation and functionality of a component, refer to "memory components," or entities embodied in a "memory" or components comprising the memory. It will be appreciated that the memory components described herein can be either volatile memory or nonvolatile memory, or can include both volatile and nonvolatile memory. Moreover, a memory component can be removable or affixed to a functional element (e.g., device, server).

Simply as an illustration, nonvolatile memory can include read only memory (ROM), programmable ROM (PROM), electrically programmable ROM (EPROM), electrically erasable ROM (EEPROM), or flash memory. Volatile memory can include random access memory (RAM), which acts as external cache memory. By way of illustration and not limitation, RAM is available in many forms such as synchronous RAM (SRAM), dynamic RAM (DRAM), synchronous DRAM (SDRAM), double data rate SDRAM (DDR SDRAM), enhanced SDRAM (ESDRAM), Synchlink DRAM (SLDRAM), and direct Rambus RAM (DRRAM). Additionally, the disclosed memory components of systems or methods herein are intended to comprise, without being limited to comprising, these and any other suitable types of memory.

Various aspects described herein can be implemented as a method, system, device, apparatus, or article of manufacture using standard programming and/or engineering techniques. In addition, various of the aspects disclosed herein also can be implemented by means of program modules or other types of computer program instructions stored in a memory device and executed by a processor, or other combination of hardware and software, or hardware and firmware. In some cases, a virtual machine (or virtual processor) can execute the program modules or the other types of computer program instructions. Such program modules or computer program instructions can be loaded onto a general purpose computer, a special purpose computer, or another type of programmable data processing apparatus to produce a machine, such that the instructions which execute on the computer or other programmable data processing apparatus create a means for implementing the functionality of disclosed herein.

The term "article of manufacture" as used herein is intended to encompass a computer program accessible from any computer-readable device, carrier, or media. For example, computer readable media can include but are not limited to magnetic storage devices (e.g., hard drive disk, floppy disk, magnetic strips, or similar), optical discs (e.g., compact disc (CD), digital versatile disc (DVD), blu-ray disc (BD), or similar), smart cards, and flash memory devices (e.g., card, stick, key drive, or similar).

What has been described above includes examples of one or more aspects of the disclosure. It is, of course, not possible to describe every conceivable combination of components or methodologies for purposes of describing these examples, and it can be recognized that many further combinations and permutations of the present aspects are possible. Accordingly, the aspects disclosed and/or claimed herein are intended to embrace all such alterations, modifications and variations that fall within the spirit and scope of the detailed description and the appended claims. Furthermore, to the extent that one or more of the terms "includes," "including," "has," "have," or "having" is used in either the detailed description or the claims, such term is intended to be inclusive in a manner similar to the term "comprising" as "comprising" is interpreted when employed as a transitional word in a claim.

## Claims

1. A method, comprising:

   receiving, by one or more processors, individually or in combination, thoracic impedance (TI) measurement signals corresponding to a subject, the TI measurement signals being time-dependent and obtained during a time interval;
   generating, by the one or more processors, individually or in combination, using the TI measurement signals, respiration signals corresponding to the subject during the time interval;
   determining, by the one or more processors, individually or in combination, over the time interval, using the respiration signals, multiple values of a ratio of an exhalation time and an inhalation time of the subject;
   monitoring, by the one or more processors, individually or in combination, over the time interval, using the multiple values, a time-dependence of the ratio; and
   identifying, by the one or more processors, individually or in combination, based on the time-dependence, a status change of a cardiopulmonary condition of the subject.

2. The method of claim 1, further comprising causing by the one or more processors, individually or in combination, a computing device to present one or more markings indicative of the status change.

3. The method of any preceding claim, wherein the cardiopulmonary condition includes at least one of airway constriction or emphysema, the method further comprising,

   determining, by the one or more processors, individually or in combination, that the time-dependence is indicative of an increase of the ratio of the exhalation time and the inhalation time of the subject; and
   identifying, by the one or more processors, individually or in combination, the status change as an exacerbation of the cardiopulmonary condition.

4.  The method of any preceding claim, wherein the cardiopulmonary condition includes at least one of airway constriction or emphysema, the method further comprising,

    determining, by the one or more processors, individually or in combination, that the time-dependence is indicative of a decrease of the ratio of the exhalation time and the inhalation time of the subject; and
    identifying, by the one or more processors, individually or in combination, the status change as an improvement of the cardiopulmonary condition.

5.  The method of any preceding claim, wherein the TI measurement signals are received from a wearable device mounted on a torso of the subject, the method further comprising causing, by the one or more processors, individually or in combination, based on the status change, the wearable device to perform a measurement cycle of thoracic impedance of the subject.

6.  The method of any preceding claim, wherein the TI measurement signals are received from a wearable device mounted on a torso of the subject, the method further comprising prompting, by the one or more processors, individually or in combination, based on the status change, the subject to cause the wearable device to perform a measurement of thoracic impedance of the subject over a measurement period.

7.  The method of any preceding claim, wherein the generating, using the TI measurement signals, the respiration signals comprises,

    filtering the TI measurement signals using a low-pass filter configured to remove frequencies exceeding a cutoff frequency within a respiration bandwidth;
    subtracting a mean value of the filtered TI measurement signals from the TI measurement signals, resulting in de-meaned TI measurement signals; and
    subtracting a moving average of the de-meaned TI measurement signals from the de-meaned TI measurement signals, resulting in the respiration signals.

8.  A computing system, comprising:

    at least one processor; and
    at least one memory device storing processor-executable instructions that, in response to execution by the at least one processor, individually or in combination, cause the computing system at least to:

    receive thoracic impedance (TI) measurement signals corresponding to a subject, the TI measurement signals being time-dependent and obtained during a time interval;
    generate, using the TI measurement signals, respiration signals corresponding to the subject during the time interval;
    determine, over the time interval, using the respiration signals, multiple values of a ratio of an exhalation time and an inhalation time of the subject;
    monitor, over the time interval, using the multiple values, a time-dependence of the ratio; and
    identify, based on the time-dependence, a status change of a cardiopulmonary condition of the subject.

9.  The computing system of claim 8, wherein the processor-executable instructions, in response to execution by the at least one processor, individually or in combination, further cause the computing system to cause a computing device to present one or more markings indicative of the status change.

10. The computing system of claims 8 or 9, wherein the cardiopulmonary condition includes at least one of airway constriction or emphysema, and wherein the processor-executable instructions, in response to execution by the at least one processor, individually or in combination, further cause the computing system to,

    determine that the time-dependence is indicative of an increase of the ratio of the exhalation time and the inhalation time of the subject; and
    identify the status change as an exacerbation of the cardiopulmonary condition.

11. The computing system of any of claims 8 to 10, wherein the cardiopulmonary condition includes at least one of airway constriction or emphysema, and wherein the processor-executable instructions, in response to execution by the at least one processor, individually or in combination, further cause the computing system to,

> determine that the time-dependence is indicative of a decrease of the ratio of the exhalation time and the inhalation time of the subject; and
> identify the status change as an improvement of the cardiopulmonary condition.

12. The computing system of any of claims 8 to 11, wherein the TI measurement signals are received from a wearable device mounted on a torso of the subject, and wherein the processor-executable instructions, in response to execution by the at least one processor, individually or in combination, further cause the computing system to cause, based on the status change, the wearable device to perform a measurement cycle of thoracic impedance of the subject.

13. The computing system of any of claims 8 to 12, wherein the TI measurement signals are received from a wearable device mounted on a torso of the subject, and wherein the processor-executable instructions, in response to execution by the at least one processor, individually or in combination, further cause the computing system to prompt, based on the status change, the subject to cause the wearable device to perform a measurement of thoracic impedance of the subject over a measurement period.

14. The computing system of any of claims 8 to 13, wherein to generate, using the TI measurement signals, the respiration signals, the processor-executable instructions, in response to execution by the at least one processor, individually or in combination, further cause the computing system at least to,

> filter the TI measurement signals using a low-pass filter configured to remove frequencies exceeding a cutoff frequency within a respiration bandwidth;
> subtract a mean value of the filtered TI measurement signals from the TI measurement signals, resulting in de-meaned TI measurement signals; and
> subtract a moving average of the de-meaned TI measurement signals from the de-meaned TI measurement signals, resulting in the respiration signals.

15. A user device, comprising:

> at least one processor; and
> at least one memory device storing processor-executable instructions that, in response to execution by the at least one processor, individually or in combination, cause the user device to perform or facilitate a method according to any one of claims 1 to 7.

**FIG. 1**

**FIG. 2A**

FIG. 2B

**FIG. 2C**

FIG. 3

FIG. 4

**FIG. 5**

FIG. 6A

EP 4 591 787 A1

**FIG. 6B**

FIG. 7

R = 0.0043

FIG. 8A

R = 0.0194

FIG. 8B

**FIG. 9A**

**FIG. 9B**

FIG. 10

FIG. 11A

**FIG. 11B**

**FIG. 12**

**FIG. 13**

1400

1410

RECEIVE THORACIC IMPEDANCE (TI) MEASUREMENT SIGNALS CORRESPONDING TO A SUBJECT, THE TI MEASUREMENT SIGNALS OBTAINED DURING A TIME INTERVAL

1420

DETERMINE, OVER THE TIME INTERVAL, USING THE TI MEASUREMENT SIGNALS, MULTIPLE VALUES OF A METRIC REPRESENTATIVE OF RESPIRATORY FUNCTION OF THE SUBJECT

1430

MONITOR, OVER THE TIME INTERVAL, USING THE MULTIPLE VALUES, A TIME-DEPENDENCE OF THE METRIC

1440

IDENTIFY, BASED ON THE TIME-DEPENDENCE OF THE METRIC, A STATUS CHANGE OF A CARDIOPULMONARY CONDITION OF THE SUBJECT

1450

DIRECT A COMPUTING DEVICE TO EXECUTE AN ACTION OR A SEQUENCE OF ACTIONS BASED ON THE STATUS CHANGE

## FIG. 14

1500

1510

GENERATE, USING THORACIC IMPEDANCE (TI) MEASUREMENT
SIGNALS DURING A TIME INTERVAL, RESPIRATION SIGNALS
CORRESPONDING TO A SUBJECT DURING THE TIME INTERVAL

1520

GENERATE, USING ACCELERATION MEASUREMENT SIGNALS
DURING THE TIME INTERVAL, MOVEMENT SIGNALS
CORRESPONDING TO MOVEMENT OF A CHEST WALL OF THE
SUBJECT DURING THE TIME INTERVAL

1530

DETERMINE, OVER THE TIME INTERVAL, USING THE RESPIRATION
SIGNALS AND THE MOVEMENT SIGNALS, MULTIPLE VALUES OF A
METRIC ASSOCIATED WITH RESPIRATION OF THE SUBJECT

METRIC IS INDICATIVE OF DELAY BETWEEN
MECHANICAL ONSET OF INSPIRATION AND ONSET OF
INSPIRATION AIRFLOW

METRIC IS REPRESENTATIVE OF RESPIRATORY EFFORT

1540

MONITOR, OVER THE TIME INTERVAL, USING THE MULTIPLE
VALUES, A TIME-DEPENDENCE OF THE METRIC ASSOCIATED
WITH RESPIRATION OF THE SUBJECT

1550

IDENTIFY, BASED ON THE TIME-DEPENDENCE, A STATUS CHANGE
OF A CARDIOPULMONARY CONDITION OF THE SUBJECT

1560

DIRECT A COMPUTING DEVICE TO EXECUTE AN ACTION OR A
SEQUENCE OF ACTIONS BASED ON THE STATUS CHANGE

FIG. 15

1600

1610

FILTER ACCELERATION MEASUREMENT SIGNALS IN A
RESPIRATION BANDWIDTH

1620

SUBTRACT RESPECTIVE MEAN VALUES OF THE
FILTERED ACCELERATION MEASUREMENT SIGNALS
FROM THE FILTERED ACCELERATION MEASUREMENT
SIGNALS, RESULTING IN DE-MEANED ACCELERATION
MEASUREMENT SIGNALS

1630

CONVERT THE DE-MEANED ACCELERATION
MEASUREMENT SIGNALS TO A SAME TIME BASE OF A
THORACIC IMPEDANCE MEASUREMENT

1640

GENERATE A PULMONARY VENTILATION SIGNAL
USING THE THORACIC IMPEDANCE MEASUREMENT

1650

DETERMINE A SATISFACTORY POLAR ANGLE OF AN
IN-PLANE ACCELERATION VECTOR FORMED BY A
FIRST DE-MEANED ACCELERATION MEASUREMENT
SIGNAL AND A SECOND DE-MEANED ACCELERATION
MEASUREMENT SIGNAL

**FIG. 16**

—1700

1710

```
RECEIVE MULTIPLE MEASUREMENT SIGNALS
CORRESPONDING TO A SUBJECT DURING A TIME
INTERVAL, EACH ONE OF THE MULTIPLE MEASUREMENT
SIGNALS IS OBTAINED ACCORDING TO A RESPECTIVE
SENSING MODALITY
```

1720

```
DETERMINE, OVER THE TIME INTERVAL, USING THE
MULTIPLE MEASUREMENT SIGNALS, MULTIPLE VALUES OF
ONE OR MULTIPLE METRICS REPRESENTATIVE OF
CARDIOPULMONARY FUNCTION OF THE SUBJECT
```

1730

```
MONITOR, OVER THE TIME INTERVAL, USING THE MULTIPLE
VALUES, RESPECTIVE TIME-DEPENDENCES OF THE ONE OR
MULTIPLE METRICS
```

1740

```
IDENTIFY, BASED ON ONE OR A COMBINATION OF THE
RESPECTIVE TIME-DEPENDENCES, A STATUS CHANGE OF A
CARDIOPULMONARY CONDITION OF THE SUBJECT
```

1750

```
DIRECT A COMPUTING DEVICE TO EXECUTE AN ACTION OR A
SEQUENCE OF ACTIONS BASED ON THE STATUS CHANGE
```

**FIG. 17**

Europäisches
Patentamt

European
Patent Office

Office européen
des brevets

# EUROPEAN SEARCH REPORT

**Application Number**

EP 25 15 4192

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/200710 A1 (THAKUR PRAMODSINGH HIRASINGH [US] ET AL) 29 June 2023 (2023-06-29) * abstract * | 1,8,15 | INV. A61B5/0535 A61B5/087 |
| A | US 2011/061647 A1 (STAHMANN JEFFREY E [US] ET AL) 17 March 2011 (2011-03-17) * abstract * | 1,8,15 | |
| A | US 2020/329977 A1 (FREEMAN JENNY E [US] ET AL) 22 October 2020 (2020-10-22) * abstract * | 1,8,15 | |
| A | US 2003/100843 A1 (HOFFMAN ANDREW [US]) 29 May 2003 (2003-05-29) * abstract * | 1,8,15 | |
| X | US 2019/167176 A1 (ANNONI ELIZABETH MARY [US] ET AL) 6 June 2019 (2019-06-06) * paragraph [0061]; table I * | 1-15 | |

**TECHNICAL FIELDS SEARCHED (IPC)**

A61B

The present search report has been drawn up for all claims

| Place of search | Date of completion of the search | Examiner |
|---|---|---|
| Berlin | 6 June 2025 | Clevorn, Jens |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 25 15 4192

This annex lists the patent family members relating to the patent documents cited in the above-mentioned European search report.
The members are as contained in the European Patent Office EDP file on
The European Patent Office is in no way liable for these particulars which are merely given for the purpose of information.

06-06-2025

| Patent document cited in search report | | | Publication date | Patent family member(s) | | | Publication date |
|---|---|---|---|---|---|---|---|
| US 2023200710 | A1 | | 29-06-2023 | CN | 113891677 | A | 04-01-2022 |
| | | | | EP | 3941341 | A1 | 26-01-2022 |
| | | | | US | 2020297230 | A1 | 24-09-2020 |
| | | | | US | 2023200710 | A1 | 29-06-2023 |
| | | | | WO | 2020190922 | A1 | 24-09-2020 |
| US 2011061647 | A1 | | 17-03-2011 | AT | E413902 | T1 | 15-11-2008 |
| | | | | EP | 1670547 | A2 | 21-06-2006 |
| | | | | EP | 2008581 | A2 | 31-12-2008 |
| | | | | US | 2005115561 | A1 | 02-06-2005 |
| | | | | US | 2011061647 | A1 | 17-03-2011 |
| | | | | WO | 2005028029 | A2 | 31-03-2005 |
| US 2020329977 | A1 | | 22-10-2020 | US | 10702166 | B1 | 07-07-2020 |
| | | | | US | 2020329977 | A1 | 22-10-2020 |
| US 2003100843 | A1 | | 29-05-2003 | NONE | | | |
| US 2019167176 | A1 | | 06-06-2019 | NONE | | | |

EPO FORM P0459

For more details about this annex : see Official Journal of the European Patent Office, No. 12/82

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63625749
- US 63625741
- US 63625787

- US 63551972
- US 63551956
- US 63551957